(19) Europäisches Patentamt
European Patent Office
Office européen des brevets

(11) Numéro de publication : **0 465 343 B1**

(12) # FASCICULE DE BREVET EUROPEEN

(45) Date de publication du fascicule du brevet : **15.06.94 Bulletin 94/24**

(51) Int. Cl.$^5$ : **A61K 31/575,** A61K 7/48, // (A61K31/575, 31:20, 31:07)

(21) Numéro de dépôt : **91401805.6**

(22) Date de dépôt : **02.07.91**

(54) **Composition pharmaceutique ou cosmétique contenant en association un rétinoïde et un stérol.**

(30) Priorité : **02.07.90 FR 9008344**

(43) Date de publication de la demande : **08.01.92 Bulletin 92/02**

(45) Mention de la délivrance du brevet : **15.06.94 Bulletin 94/24**

(84) Etats contractants désignés : **AT BE CH DE DK ES FR GB GR IT LI NL SE**

(56) Documents cités :
**EP-A- 0 337 890**
**EXP. CELL RESEARCH, vol. 171, 1987; pp. 426-435**

(73) Titulaire : **CENTRE INTERNATIONAL DE RECHERCHES DERMATOLOGIQUES GALDERMA - CIRD GALDERMA**
**635, route des Lucioles,**
**Sophia Antipolis**
**F-06565 Valbonne (FR)**

(72) Inventeur : **Reichert, Uwe**
**Villa Valrose,**
**Pont du Loup - Gourdon**
**F-06620 . Le Bar S/Loup (FR)**
Inventeur : **Schmidt, Rainer**
**Chemin du Figueret**
**F-06620 Gourdon (FR)**
Inventeur : **Shroot, Braham,**
**18 Bis, Boulevard Albert Ier**
**F-06600 - Antibes (FR)**

(74) Mandataire : **Tonnellier, Jean-Claude et al**
**Cabinet Nony & Cie.**
**29, rue Cambacérès**
**F-75008 Paris (FR)**

EP 0 465 343 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet une composition pharmaceutique ou cosmétique à effet synergétique, destinée notamment à traiter les troubles de la kératinisation épidermique, les troubles de la prolifération épidermique ou épithéliale, et/ou les troubles de la fonction sébacée, ladite composition comprenant, en association, au moins un rétinoïde et au moins un stéroïde inhibiteur de la biosynthèse du cholestérol.

On sait que les agents contrôlant la différenciation cellulaire, tels que les rétinoïdes, sont largement utilisés ou préconisés pour traiter les désordres de la kératinisation. Ces rétinoïdes ont notamment la propriété d'inhiber la formation des enveloppes cornées (EC) de l'épiderme, c'est-à-dire la phase ultime de la différenciation des kératinocytes. Le mécanisme d'action biologique mis en jeu est connu : il s'agit d'une inhibition de l'expression de la transglutaminase membranaire (TGm) qui est une enzyme essentielle dans la synthèse des EC ; voir par exemple FEBS LETTERS Vol 258 n°1 p.35-38 (1989), Vol 229 n°1 p.193-196 (1988) et Vol 186 n°2 p.201-204 (1985), et J. Invest. Dermatol Vol 90 n°4 p.472-474 (1988).

On sait par ailleurs que le 25-hydroxy cholestérol est également capable d'inhiber la synthèse des EC ; voir notamment Exp. Cell. Res. 171 p.426-435 (1987).

On a maintenant découvert que l'inhibition de la synthèse des enveloppes cornées par le 25-hydroxy cholestérol résulte d'un mécanisme biochimique différent : il s'agit d'une inhibition de la biosynthèse du cholestérol.

On a également découvert que les compositions contenant, en association, à la fois un rétinoïde capable d'inhiber l'expression de la TGm et un stérol capable d'inhiber la biosynthèse du cholestérol présente un effet synergétique en ce qui concerne l'inhibition de la synthèse des EC.

Ces deux types de composés agissant par deux voies biochimiques indépendantes, on devait normalement s'attendre, en les associant, à obtenir une activité résultante inhibitrice de la synthèse des EC égale à l'addition des activités obtenues avec les composés testés séparément. Or on a découvert, de façon surprenante, que l'association mentionnée ci-dessus provoque une synergie : le niveau d'inhibition obtenu est très nettement supérieur à l'addition des inhibitions dues aux constituants de l'association pris séparément.

La présente invention a pour objet une composition synergétique résultant de l'association de deux agents contrôlant la différenciation cellulaire par des mécanismes d'action différents, pour traiter ou corriger les troubles de la kératinisation épidermique, tout autre trouble ou tout autre défaut ou excès fonctionnel de la prolifération épidermique ou épithéliale et/ou les troubles de la fonction sébacée, accompagnés ou non d'une composante inflammatoire et/ou immunoallergique. La composition de l'invention permet par exemple de traiter les maladies de dégénérescence du tissu conjonctif, les tumeurs bénignes ou malignes, de lutter contre le vieillissement cutané, de favoriser la cicatrisation, ou encore d'améliorer l'aspect de la peau des personnes présentant des troubles de la kératinisation.

L'association selon la présente invention trouve également une application dans le domaine ophtalmologique, notamment dans le traitement des cornéopathies.

L'utilisation de cette association présente notamment l'avantage de permettre d'obtenir une activité suffisante tout en diminuant nettement le taux d'agent contrôlant la différenciation cellulaire et par conséquent en limitant, ou même en supprimant, les effets secondaires dus à cet agent. On peut ainsi utiliser par exemple un composé ayant une activité de type rétinoïde beaucoup moins puissante, et donc moins toxique, que celle de l'acide rétinoïque, et obtenir cependant, grâce à la synergie, une activité équivalente.

La composition pharmaceutique ou cosmétique à effet synergétique de l'invention comprend donc en association :

- au moins un rétinoïde capable d'inhiber l'expression de la TGm,
- et au moins un stérol inhibiteur de la biosynthèse du cholestérol.

Les rétinoïdes constituent une classe de composés connue, qui se compose de la vitamine A, de ses dérivés naturels et de tous les dérivés synthétiques ayant une affinité pour les récepteurs nucléaires de l'acide rétinoïque ; voir par exemple Nouv. Dermatol. 9,1, 3-6 (1990).

On entend ici par "rétinoïdes" les composés qui répondent à cette définition, et en particulier l'acide rétinoïque et ses dérivés, et aussi divers analogues qui seront mentionnés ci-après.

Les rétinoïdes utilisables dans la composition de la présente demande sont ceux qui sont capables d'inhiber l'expression de la TGm, par exemple selon le test décrit par S. MICHEL et Coll. dans Models Dermatol., MAIBACH et LOWE Eds, Karger, Basel Vol 4, p.40-44 (1989).

Parmi les rétinoïdes ou produits ayant une activité du type rétinoïde, utilisables dans les compositions de l'invention, on citera notamment les dérivés suivants, ainsi que leurs esters et leurs amides :

- Acide rétinoïque all-trans ou 13-cis,
- La vitamine A ou rétinol, ainsi que ses esters tels que l'acétate, le propionate ou le palmitate de rétinol,
- L'aldéhyde de vitamine A ou rétinal,

- Acide (all-E)-9-(4-méthoxy-2,3,6-triméthylphényl)-3,7-diméthyl-2,4,6,8-nonatétraénoïque (Acitretin),
- Acide (E,E,E)-7-(2,3-dihydro-1,1,3,3-tétraméthyl-1H-indèn-5-yl)-3,7-diméthyl-2,4,6-octatriènoïque,
- Acide (E,E,E)-7-(5,6,7,8-tétrahydro-5,5,8,8-tétraméthyl-2-naphtalènyl)-3,7-diméthyl-2,4,6-octatriènoïque,
- Acide (E)-4-[(2,3-dihydro-1,1,3,3-tétraméthyl-1H-indèn-5-yl)-1-propènyl] benzoïque,
- Acide (E)-4-[2-(5,6,7,8-tétrahydro-5,5,8,8-tétraméthyl-2-naphtalènyl)-1-propènyl] benzoïque,
- Acide (E)-4-[2-(5,6,7,8-tétrahydro-3-méthyl-5,5,8,8-tétraméthyl-2-naphtalènyl)-1-propènyl] benzoïque,
- Acide 2-(5,6,7,8-tétrahydro-5,5,8,8-tétraméthyl-2-naphtyl)-6-naphtalène carboxylique,
- Acide (E)-4-[2-(5,6,7,8-tétrahydro-5,5,8,8-tétraméthyl-2-naphtalènyl)-1-propènyl] benzène sulfonique,
- Acide (E,E)-4-[2-méthyl-4-(2,6,6-triméthyl-1-cyclohexèn-1-yl)-1,3-butadiènyl] benzoïque,
- Acide (E,E)-4-[4-méthyl-6-(2,6,6-triméthyl-1-cyclohexèn-1-yl)-1,3,5-hexatrienyl] benzoïque,
- Acide (E)-6-[2-(2,6,6-triméthyl-1-cyclohexèn-1-yl)-éthènyl]-2-naphtalènecarboxylique,
- Acide (E,E)-5-[2-méthyl-4-(2,6,6-triméthyl-1-cyclohexèn-1-yl)-1,3-butadiènyl]-2-thiophène carboxylique,
- Acide (E)-4-[2-(5,6,7,8-tétrahydro-8,8-diméthyl-2-naphtalènyl)-1-propènyl]benzoïque,
- Acide 4-[(5,6,7,8-tétrahydro-5,5,8,8-tétraméthyl-2-naphtalènyl)-éthynyl]benzoïque,
- Acide 4-(5,6,7,8-tétrahydro-5,5,8,8-tétraméthyl-2-naphtalènylcarbamoyl) benzoïque,
- Acide 4-(5,6,7,8-tétrahydro-5,5,8,8-tétraméthyl-2-naphtamido) benzoïque,
- Acide (E)-4-[3-(3,5 di-tert-butylphényl)-3-oxo-1-propènyl] benzoïque,
- Acide 6-[(5,6,7,8-tétrahydro-5,5,8,8-tétraméthyl-2-naphtalènyl) éthynyl]3-pyridine carboxylique,

On peut également citer les dérivés suivants :

- les rétinoïdes hétérocycliques aromatiques décrits dans le brevet FR 85.13747 (FR 2 570 377) tels que :
  - l'acide 2-(5,6,7,8-tétrahydro-5,5,8,8-tétraméthyl-2-naphtyl)-6-benzo(b) thiophène carboxylique,
  - l'acide 2-(5,6,7,8-tétrahydro-5,5,8,8-tétraméthyl-2-naphtyl)-6-benzo(b) furanecarboxylique,
  - l'acide 2-(5,6,7,8-tétrahydro-5,5,8,8-tétraméthyl-2-naphtyl)-6-indole carboxylique,
  - l'acide 2-[3-(1-adamantyl)-4-méthoxyphényl]-5 benzimidazole carboxylique,
  - l'acide 2-(5,6,7,8-tétrahydro-5,5,8,8-tétraméthyl-2-naphtyl)-5-benzimidazole carboxylique.
- les rétinoïdes aromatiques polycycliques décrits dans la demande de brevet EP 0 210 929 tels que :
  - l'acide 4-(5,6,7,8-tétrahydro-5,5,8,8-tétraméthyl-2-anthracényl) benzoïque.
- les rétinoïdes benzonaphtaléniques décrits dans la demande de brevet EP 0 199 636, comme par exemple :
  - l'acide 6-[3-(1-adamantyl)-4-méthoxyphényl]-2-naphtoïque.
- les rétinoïdes benzamido aromatiques décrits dans la demande de brevet EP 0 232 199 comme par exemple :
  - l'acide 4-[3-(1-adamantyl)-4-méthoxybenzamido]benzoïque.
- les rétinoïdes benzoyloxythio aromatiques décrits dans la demande de brevet EP 0 325 540, tels que :
  - l'acide 4-[3-(1-adamantyl)-4-méthoxy benzoylthio]benzoïque ou
  - l'acide 4-[3-(1-adamantyl) 4-méthoxy benzoyloxy]benzoïque
- les rétinoïdes bicycliques naphtaléniques décrits dans la demande de brevet EP 0 220 118, par exemple :
  - l'acide 2- (5,6,7,8-tétrahydro-5,5,8,8-tétraméthyl-2-naphtyl) 6-carbonyl naphtalène carboxylique.
- les rétinoïdes bicycliques aromatiques décrits dans le brevet FR 87-06152 (2 614 618) comme par exemple :
  - l'acide p-(5,6,7,8-tétrahydro-5,5,8,8-tétraméthyl-2-1H-benz[f]indolyl)benzoïque.
- les rétinoïdes bicycliques aromatiques décrits dans le brevet FR 86 10423 (2 601 670), par exemple :
  - l'acide trans-(5,5,8,8-tétraméthyl-5,6,7,8 tétrahydro-2-napthtyl) -4-carbonyl-$\alpha$-méthyl cinnamique.
- les rétinoïdes esters bi-aromatiques décrits dans la demande de brevet EP 90-402072, de formule générale :

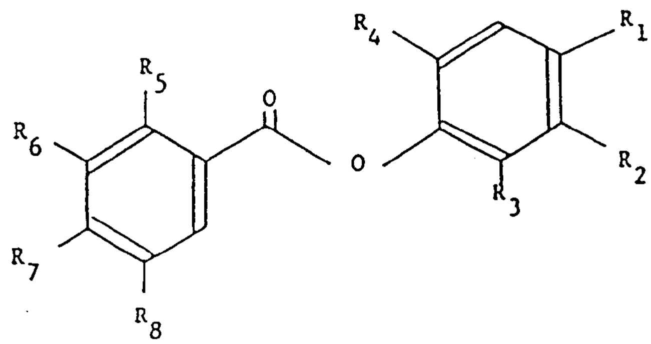

dans laquelle:

$R_1$ représente un atome d'hydrogène, le groupe OH, le radical $-CH_3$, $-CH_2OH$, $-CH(OH)CH_3$, $-COOR_9$,

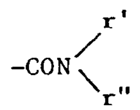

ou $SO_2R_{10}$,

$R_9$ représente un atome d'hydrogène, un radical alkyle ayant de 1 à 6 atomes de carbone ou un radical mono ou polyhydroxyalkyle,

$R_{10}$ représentant le groupe OH, un radical alkyle ayant de 1 à 6 atomes de carbone ou le radical

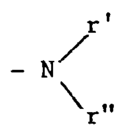 ,

r′ et r″ représentant un atome d'hydrogène, un radical alkyle ayant de 1 à 6 atomes de carbone, aryle, aralkyle, mono ou polyhydroxyalkyle, ou r′ et r″ pris ensemble forment un hétérocycle,

$R_2$ représente un atome d'hydrogène, un radical alkyle ayant de 1 à 6 atomes de carbone, le radical $OR_9$ , un atome de fluor ou le radical $-CF_3$,

$R_3$ , $R_4$ et $R_5$ représentent un atome d'hydrogène, un atome de fluor, le groupe OH, le radical $-CH_3$, $-OCH_3$, $-CF_3$,-COOH ou $-CH_2OH$,

$R_5$ et $R_8$ représentent un atome d'hydrogène, un radical alkyle $\alpha$ -substitué ayant de 3 à 15 atomes de carbone, un radical alkyle $\alpha$ -$\alpha'$ disubstitué ayant de 4 à 12 atomes de carbone, un radical cycloalkyle ayant de 3 à 12 atomes de carbone, un radical cycloalkyle mono ou polycyclique ayant de 5 à 12 atomes de carbone dont le carbone de liaison est trisubstitué, le radical $-SR_{11}$, $-SO_2R_{11}$ ou $-SOR_{11}$.

$R_{11}$ représentant un radical alkyle ayant de 1 à 6 atomes de carbone ou un radical cycloalkyle, $R_6$ et $R_8$ ne pouvant pas simultanément représenter un atome d'hydrogène,

$R_7$ représente un atome d'hydrogène, un radical alkyle ayant de 1 à 6 atomes de carbone, un radical alkényle, un radical alkényloxy, le radical $OR_{12}$, $SR_{13}$, $SOR_{14}$ ou $SO_2R_{14}$,

$R_{12}$ représentant un atome d'hydrogène, un radical alkyle ayant de 1 à 6 atomes de carbone, un radical alkényle, un radical mono ou polyhydroxy alkyle ou le radical $-(CH_2)_n-COR_{15}$,

n étant 0,1 ou 2 et $R_{15}$ représentant un atome d'hydrogène, le groupe OH, un radical alkyle ayant de 1 à 6 atomes de carbone ou un radical alkoxy ayant de 1 à 6 atomes de carbone.

$R_{13}$ représentant un atome d'hydrogène, un radical alkyle ayant de 1 à 6 atomes de carbone ou un radical aralkyle,

$R_{14}$ représentant le groupe OH, un radical alkyle ayant de 1 à 6 atomes de carbone ou un radical aralkyle, sous réserve que lorsque $R_1$ représente $-CH_2OH$, $-CH(OH)CH_3$, $-COOR_9$ ou

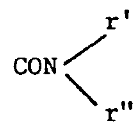

et $R_2$ représente un atome d'hydrogène alors :

(i) soit $R_3$ et $R_4$ sont différents d'un atome d'hydrogène ou du radical $-CH_3$ ,

(ii) soit $R_7$ est différent du radical $OR_{12}$ et $R_6$ ou $R_8$ est un radical cycloalkyle ayant plus de 7 atomes de carbone,

(iii) soit $R_7$ représente le radical $OR_{12}$ , mais $R_6$ et $R_8$ sont alors différents d'un atome d'hydrogène,

(iv) soit $R_7$ représente le radical $OR_{12}$ , mais alors $R_5$ est différent d'un atome d'hydrogène,

et en particulier :

- l'acide 4- [3-(1-adamantyl)-4-méthoxybenzoyloxy] -2-fluorobenzoïque.
- l'acide 4- [3-(1-adamantyl)-4-méthoxybenzoyloxy] -2-methylbenzoïque.
- l'acide 4- [3-(1-adamantyl)-4-méthoxybenzoyloxy] -2-hydroxybenzoïque.
- l'acide 4- [5-(1-adamantyl)-2-fluoro-4-méthoxybenzoyloxy] benzoïque.
- l'acide 4- [3,5-di-tert-butyl-4-hydroxybenzoyloxy] benzoïque
- l'acide 4- [3-(1-adamantyl)-4-vinylbenzoyloxy] benzoïque
- l'acide 4- [3-(1-adamantyl)-4-éthylbenzoyloxy] benzoïque
- l'acide 4- [3-(1-adamantyl)-4-allyloxybenzoyloxy] benzoïque
- l'acide 4- [3-(1-adamantyl)-4-méthylthiobenzoyloxy] benzoïque

- les rétinoïdes bi-aromatiques décrits dans la demande luxembourgeoise 87821 de formule générale I :

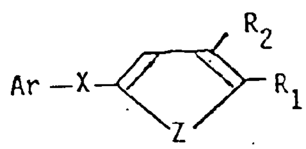

(I)

dans laquelle :

Ar représente soit le radical

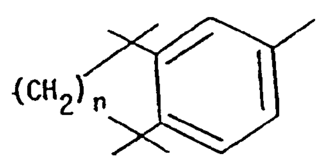

(II)

avec n = 1 ou 2

soit le radical

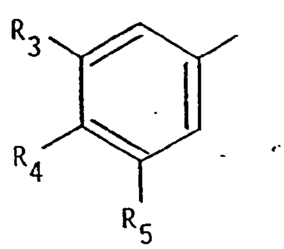

(III)

$R_3$ et $R_5$ représentant un atome d'hydrogène, le groupe OH, un radical alcoxy ayant de 1 à 6 atomes de carbone, un radical alkyle $\alpha$-substitué ayant le 3 à 12 atomes de carbone ou un radical alkyle $\alpha,\alpha'$-disubstitué ayant de 4 à 12 atomes de carbone, un radical cycloalkyle ayant de 3 à 12 atomes de carbone, un radical cycloalkyle mono ou polycyclique ayant de 5 à 12 atomes de carbone dont le carbone de liaison est trisubstitué,

$R_4$ représentant un atome d'hydrogène, le groupe OH, un radical alcoxy ayant de 1 à 6 atomes de carbone, un radical alkyle $\alpha$-substitué ayant de 3 à 12 atomes de carbone, un radical alkyl $\alpha,\alpha'$-disubstitué ayant

de 4 à 12 atomes de carbone, un radical cycloalkyle ayant de 3 à 12 atomes de carbone, un radical cycloalkyle mono ou polycyclique ayant de 5 à 12 atomes de carbone dont le carbone de liaison est trisubstitué, un radical monohydroxyalkyle, un radical polyhydroxyalkyle, un atome de fluor, un atome de chlore, le groupe SH, le groupe $SR_6$, le groupe $SOR_6$, le groupe $SO_2R_6$, un radical alkényle ayant de 2 à 6 atomes de carbone, ou un radical alkényloxy ayant de 2 à 6 atomes de carbone,

$R_6$ représentant un radical alkyle inférieur,

$R_1$ représente un atome d'hydrogène, le groupe OH, le radical $-CH_3$, le radical $-CH_2OH$, le radical $-COR_7$, le radical $-CH(OH)CH_3$, le radical $-CH_2OCOR_6$, le radical $-SO_2R_9$, le radical $-SOR_9$ ou le radical $-SR_9$,

$R_7$ représentant un atome d'hydrogène, le groupe OH, le radical $-OR_{10}$, le radical -N(r'r''), un radical alkyle inférieur, un radical monohydroxyalkyle, un radical polyhydroxyalkyle ou le reste d'un sucre,

$R_{10}$ représentant un radical alkyle ayant de 1 à 12 atomes de carbone ou un radical alkényle ayant de 2 à 12 atomes de carbone,

r' et r'' identiques ou différents, représentent un atome d'hydrogène, un radical alkyle inférieur, un radical aryle, un radical aralkyle, un reste d'aminoacide, un reste de sucre, un reste de sucre aminé, ou un hétérocycle, où r' et r'' pris ensemble forment un hétérocycle,

$R_8$ représentant un radical alkyle linéaire ou ramifié saturé ou insaturé ayant de 1 à 20 atomes de carbone ou le reste d'un sucre,

$R_9$ représentant le groupe OH, un radical alkyle inférieur ou le radical -N(r'r''),

$R_2$ représentant un atome d'hydrogène, le groupe OH, un radical alkyle inférieur, un radical alcoxy ayant de 1 à 6 atomes de carbone, un atome de fluor, un atome de chlore, le groupe $CF_3$, le groupe $COR_7$, le groupe $CH_2OH$ ou le groupe $CH_2OR_6$,

Z représente un atome d'oxygène ou de soufre, le radical divalent $-CH=CR_{11}-$, le radical divalent -N=CH- ou le radical divalent $-N=CR_6-$,

$R_{11}$ représentant un atome d'hydrogène, le groupe OH, un radical alkyle inférieur, un radical alcoxy ayant de 1 à 6 atomes de carbone, un atome de fluor, un atome de chlore, ou le groupe $CF_3$,

X est un radical divalent qui peut être lu de gauche à droite ou inversement, choisi dans le groupe constitué par :

(i) 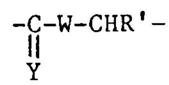

R' représentant un atome d'hydrogène, le radical $-CH_3$,

W représentant un atome d'oxygène ou de soufre ou le groupe -NR',

Y représentant un atome d'oxygène ou encore un atome de soufre lorsque W représente le groupe -NR',

(ii) 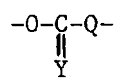

Q représentant un atome d'oxygène ou -NR',

Y représentant un atome d'oxygène ou encore un atome de soufre lorsque Q représente, le groupe -NR',

(iii) 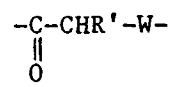

(iv) 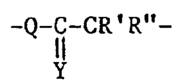

R'' représentant un atome d'hydrogène, le radical $-CH_3$, le groupe OH, un atome de fluor ou un

atome de chlore, ou

R′ et R″ pris ensemble forment un radical méthano ($=CH_2$) ou un radical oxo (=O),

(v) 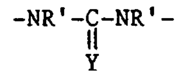

Y représentant un atome d'oxygène ou un atome de soufre, et les sels des composés de formule (I) lorsque $R_1$ représente une fonction acide carboxylique et les isomères optiques desdits composés de formule (I).

et en particulier :

- l'acide 4-(5,6,7,8-tétrahydro-5,5,8,8-tétraméthyl-2-naphtylglyoxyloyloxy) benzoïque
- l'acide 4-(5,6,7,8-tétrahydro-5,5,8,8-tétraméthyl-2-naphtoyloxyméthyl) benzoïque
- l'acide 4-(3,5-di-tert-butyl-4-hydroxybenzoyloxyméthyl) benzoïque
- l'acide 4-(3-tert-butyl-4-méthoxybenzoyloxyméthyl) benzoïque
- l'acide 5-(5,6,7,8-tétrahydro-5,5,8,8-tétraméthyl-2-naphtoyloxyméthyl)-2-thiophènecarboxylique
- l'acide 4-(5,6,7,8-tétrahydro-5,5,8,8-tétraméthyl-2-naphtoylméthyloxy) benzoïque
- l'acide 4- [1-(5,6,7,8-tétrahydro-5,5,8,8-tétraméthyl-2-naphtoyloxy) éthyl] benzoïque
- l'acide 4-[[(5,6,7,8-tétrahydro-5,5,8,8-tétraméthyl-2-naphtyl) éthyloxy] carbonyl]benzoïque
- l'acide 4-(5,6,7,8-tétrahydro-5,5,8,8-tétraméthyl-2-naphtoylméthylamino) benzoïque
- l'acide 4-(5,6,7,8-tétrahydro-5,5,8,8-tétraméthyl-2-naphtoyl formamido) benzoïque
- l'acide 4-(α-hydroxy-5,6,7,8-tétrahydro-5,5,8,8-tétraméthyl-2-naphtylacétamido) benzoïque
- l'acide 4-(α-fluoro-5,6,7,8-tétrahydro-5,5,8,8-tétraméthyl-2-naphtylacétamido) benzoïque.

Divers schémas réactionnels peuvent être envisagés pour l'obtention de ces composés de formule (I), dans le cas où Y est un atome d'oxygène.

On peut citer les schémas réactionnels suivants :

Dans le cas où X est soit (i) soit (iv)

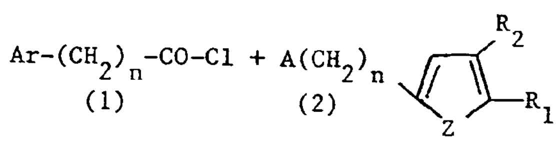

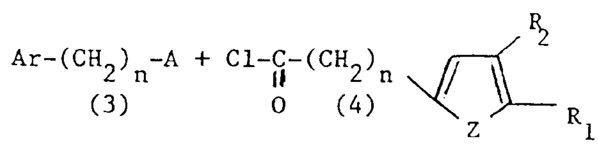

$n = 0,1$

A = $-NH_2$, $-OH$, $-SH$

L'étape principale de cette préparation consiste à faire réagir en milieu anhydre, dans un solvant organique tel que le tétrahydrofuranne ou le chlorure de méthylène contenant une amine tertiaire (triéthylamine) ou de la pyridine ou un hydrure alcalin (hydrure de sodium), une forme activée d'un acide aromatique substitué ou d'un acide arylacétique substitué par exemple un chlorure d'acide (1) ou (4) ou un anhydride mixte, sur un composé aromatique porteur d'une fonction hydroxy ou amino ou thiol (2) ou (3), la réaction étant conduite à température ambiante et sous agitation.

Lorsque $R_1$ représente le radical -COOH, les composés sont préparés en protégeant $R_1$ par un groupe protecteur de type allylique, benzylique ou tert-butylique.

Le passage à la forme libre peut être effectué :

- dans le cas d'un groupe protecteur allylique, au moyen d'un catalyseur tel que certains complexes de

métaux de transition en présence d'une amine secondaire,

- dans le cas d'un groupe protecteur benzylique, par débenzylation en présence d'hydrogène, au moyen d'un catalyseur tel que le palladium sur charbon,
- dans le cas d'un groupe protecteur tert-butylique au moyen d'iodure de triméthylsilyle.

Dans le cas où X est soit (ii) soit (v)

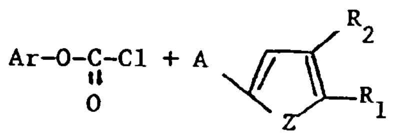

(1) (2)

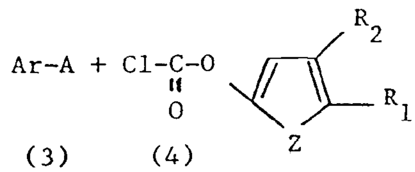

(3) (4)

A = { - $NH_2$ ; - OH }

L'étape principale de cette préparation consiste à faire réagir en milieu anhydre dans un solvant organique tel que le chlorure de méthylène contenant une amine tertiaire (triéthylamine) ou de la pyridine un chloroformiate (1) ou (4) préparé par exemple à partir d'un dérivé hydroxyaryle et du chloroformiate de trichlorométhyle ou du phosgène, avec un composé aromatique porteur d'une fonction hydroxy ou amino (2) ou (3). La réaction étant conduite à température ambiante et sous agitation.

Dans le cas où $R_1$ représente le radical -COOH, les composés sont préparés de préférence en protégeant $R_1$ par un groupe protecteur benzylique. Le passage à la forme libre s'effectue alors par débenzylation en présence d'hydrogène, au moyen d'un catalyseur tel que le palladium sur charbon.

Dans le cas où X est (iii) et si Z n'est pas un atome d'oxygène,

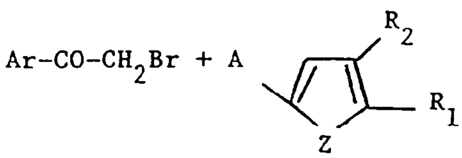

A = { $-NH_2$ ; $-OH$ ; $-SH$ }

L'étape principale de cette préparation consiste à faire réagir en présence de carbonate de potassium ou d'un hydrure alcalin (hydrure de sodium) ou par transfert de phase en utilisant par exemple le bromure de tétrabutylammonium comme sel d'ammonium quaternaire, une alpha-bromocétone aromatique avec un composé aromatique porteur d'une fonction hydroxy ou amino ou thiol en para du radical $R_1$.

Dans le cas où $R_1$ représente le radical -COOH, les composés sont préparés de préférence en protégeant $R_1$ par un groupe protecteur allylique. Le passage à la forme libre s'effectue au moyen d'un catalyseur tel que le tetrakis (triphénylphosphine) palladium (O) en présence d'une amine secondaire (morpholine).

Les acides ainsi obtenus peuvent être convertis de manière connue en chlorure d'acide correspondant qui, traité par un alcool ($R_6OH$) ou une amine HN(r')(r") donne l'ester ou l'amide correspondant.

- les rétinoïdes polycycliques aromatiques décrits dans la demande de brevet FR 91-05394, de formule gé-

nérale (IV) :

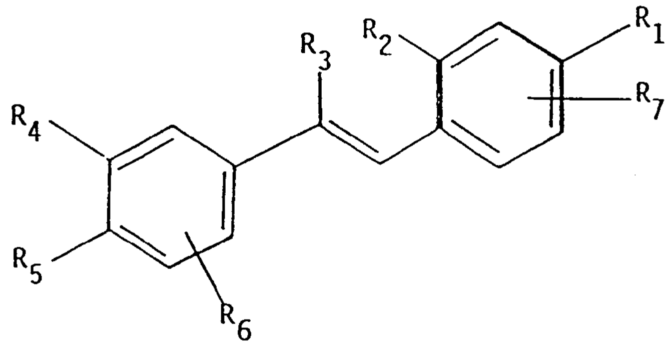

(IV)

dans laquelle :

$R_1$ représente

(i) un atome d'hydrogène,

(ii) le radical $-CH_3$,

(iii) le radical $-CH_2-O-R_8$,

$R_8$ représentant un atome d'hydrogène ou un radical alkyle inférieur,

(iv) un radical $-OR_8$,

(v) un radical

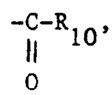

$R_{10}$ représentant :

(a) un atome d'hydrogène,

(b) un radical

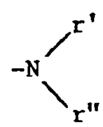

r' et r'' représentant un atome d'hydrogène, un radical alkyle inférieur, un radical mono ou polyhydroxyalkyle, un radical aryle éventuellement substitué ou un reste d'amino acide ou de sucre ou encore pris ensemble forment un hétérocycle,

(c) un radical $-OR_{11}$

$R_{11}$ représentant un atome d'hydrogène, un radical alkyle, linéaire ou ramifié, ayant de 1 à 20 atomes de carbone, un radical mono ou polyhydroxyalkyle, un radical aryle ou aralkyle éventuellement substitué(s) ou un reste de sucre ou un reste d'amino acide,

(vi) un radical $-S(O)_t R_8$,

t étant 0, 1 ou 2 et $R_8$ étant tel que défini ci-dessus, et

(vii) un radical

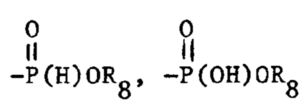

ou

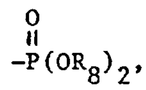

$R_8$ étant tel que défini ci-dessus,

$R_2$ représente un atome d'hydrogène,

$R_3$ représente un atome d'hydrogène, un radical aryle, un radical aralkyle ou un radical alkyle inférieur éventuellement substitué par un hydroxyle, par un alcoxy inférieur ou par un radical

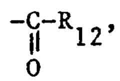

$R_{12}$ représentant un atome d'hydrogène, un radical alkyle inférieur, un radical hydroxyle, un radical alcoxy inférieur ou un radical

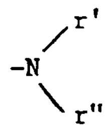

r' et r'' ayant les mêmes significations que ci-dessus,

ou $R_2$ et $R_3$ pris ensemble forment, avec le noyau benzénique, un cycle naphtalénique,

$R_4$ représente un radical alkyle linéaire ou ramifié ayant de 1 à 15 atomes de carbone ou un radical cycloaliphatique,

$R_5$ représente le radical $-(CH_2)_n-R_{13}$, le radical $-CH=CH-(CH_2)_n-R_{13}$, ou le radical $-O(CH_2)_mR_{14}$

n étant 0 ou 1 à 6,

m étant 1 à 6

$R_{13}$ représentant le radical

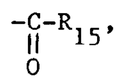

un radical monohydroxyalkyle ou un radical polyhydroxyalkyle dont les hydroxyles sont éventuellement protégés sous forme de méthoxy ou d'acétoxy, un radical alkyle inférieur epoxydé ou le radical

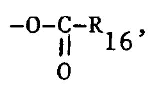

$R_{15}$ représentant le radical $OR_{16}$ ou le radical

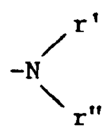

$R_{16}$ représentant un atome d'hydrogène, un radical alkyle inférieur, un radical aryle ou un radical aralkyle,

$R_{14}$ représentant un radical hydroxyle lorsque $m \geqq 2$, un radical monohydroxyalkyle, un radical polyhydroxyalkyle, le radical

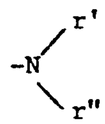

le radical

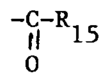

ou un radical alkényle mono ou polyhydroxylé ayant de 2 à 10 atomes de carbone, ou lorsque $R_2$ et $R_3$ ne sont pas pris ensemble, m peut être 0 et/ou $R_{14}$ peut représenter un atome d'hydrogène ou un radical alkyle inférieur, $R_6$ et $R_7$ représentent un atome d'hydrogène, un atome d'halogène, un radical alkyle inférieur ou le radical $-OR_{16}$,

$R_5$ et $R_6$ peuvent en outre former un cycle méthylène dioxy lorsque $R_6$ est en position 3 du noyau benzénique,

et les sels des composés de formule (IV)lorsque $R_1$ ou $R_{13}$ représente une fonction acide carboxylique ou lorsque $R_{14}$ représente une fonction amine et les analogues chiraux desdits composés de formule (IV), et en particulier :

- l'acide 6- [3-(1-adamantyl)-4-(2,3-dihydroxypropyloxy) phényl]-2-naphtoïque
- l'acide 6-[3-(1-adamantyl)-4-(3-hydroxypropyloxy) phényl]-2-naphtoïque
- l'acide 6-[3-(1-adamantyl)-4-acétoxyméthylphényl]-2-naphtoïque
- l'acide 6-[3-(1-adamantyl)-4-méthoxycarbonylphényl]-2-naphtoïque
- l'acide 6-[3-(1-adamantyl)-4-méthoxycarbonyléthylphényl]-2-naphtoïque
- l'acide 6-[3-(1-adamantyl)-4-(2-hydroxypropyl) phényl]-2-naphtoïque.

Les composés de formule (IV) pour lesquels $R_2$ et $R_3$ pris ensemble forment avec le noyau benzénique un cycle naphtalénique sont obtenus par une réaction de couplage entre un dérivé halogéné (1) et un dérivé halogéné de formule (2) :

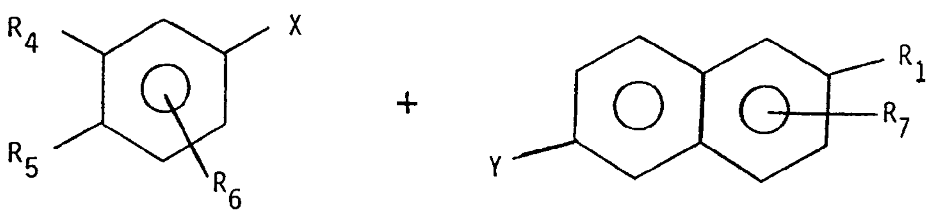

(1) (2)

X et Y représentant un atome de chlore, de brome ou d'iode.

Dans un premier temps, l'halogènure (1) est converti en un lithien, magnesien ou zincique puis est couplé au dérivé (2) en présence d'un catalyseur au nickel ou au palladium, selon les conditions de couplage biaryl décrites par E. Negishi et al., J. Org. Chem. (1977) 42, 1821.

Les composés de formule (IV) pour lesquels $R_2$ et $R_3$ ne sont pas pris ensemble peuvent être obtenus par le schéma réactionnel suivant en mettant en jeu une réaction de Wittig ou Horner-Emmons.

Dans ces réactions d'oléfination, l'isomère géométrique de configuration E peut aussi être obtenu par conversion sous irradiation sous lumière UV de l'isomère de configuration géométrique Z.

Dans ces formules $R_1$, $R_4$, $R_6$ et $R_7$ ont les mêmes significations que celles données ci-dessus pour la formule générale ou en sont des dérivés convenablement protégés pour être compatibles avec les conditions de couplage. En particulier, le substituant $R_6$ est un phénol protégé sous forme de tert-butyldiméthylsilyloxy ou un radical alcoxy.

Le dérivé obtenu est ensuite converti en phénol par déprotection au niveau du substituant $R_6$ du groupe TBDMS ou alcoxy puis est traité selon l'une des deux voies mentionnées ci-dessous :

- traitement du phénol ainsi obtenu par un hydrure métallique que l'on fait réagir sur un halogénure,
- conversion du phénol ainsi obtenu en triflate puis substitution nucléophile en présence d'un catalyseur

au palladium.

- les rétinoïdes bi-aromatiques dérivés d'un motif salicylique décrits dans la demande de brevet FR 91-05747 de formule générale (V) suivante :

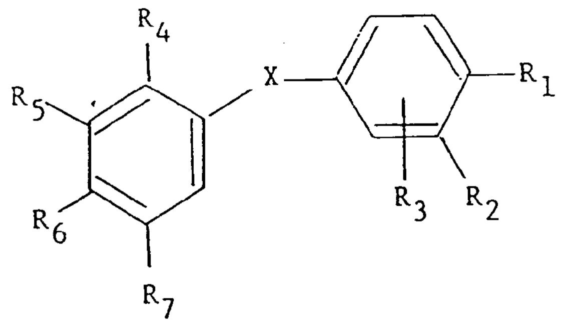

(V)

dans laquelle :

$R_1$ représente le radical - $CH_3$, le radical - $CH_2OH$, le radical - $COR_8$, ou le radical - $CH_2OCOR_9$,

$R_8$ représentant un atome d'hydrogène, OH, - $OR_{10}$,

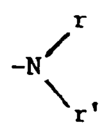

,

ou un radical alkyle inférieur,

$R_{10}$ représentant un radical alkyle ayant de 1 à 20 atomes de carbone, un radical alkényle ayant de 2 à 20 atomes de carbone, un radical aryle ou aralkyle,

r et r', identiques ou différents, représentant un atome d'hydrogène, un radical alkyle inférieur, un radical aryle, un radical aralkyle, un reste d'aminoacide, un reste de sucre ou un hétérocycle ou r et r' pris ensemble formant un hétérocycle,

$R_9$ représentant un radical alkyle ayant de 1 à 20 atomes de carbone, un radical alkényle ayant de 2 à 20 atomes de carbone ou un reste de sucre,

$R_2$ et $R_3$ représentent - $OR_{11}$ ou - $OCOR_{11}$

$R_{11}$ représentant un atome d'hydrogène, un radical alkyle inférieur, un radical fluoroalkyle ayant de 1 à 6 atomes de carbone et de 3 à 7 atomes de fluor, un radical aryle ou un radical aralkyle,

$R_3$ pouvant représenter en outre un atome d'hydrogène,

$R_4$ représente un atome d'hydrogène, OH, un radical alkyle inférieur, un radical alkoxy ayant de 1 à 6 atomes de carbone, un atome de fluor, de chlore ou le groupe - $CF_3$,

$R_5$ et $R_7$ représentent un atome d'hydrogène, OH, un radical alkoxy ayant de 1 à 6 atomes de carbone, un radical alkyle $\alpha$-substitué ayant de 3 à 12 atomes de carbone ou un radical alkyle $\alpha$-$\alpha'$-disubstitué ayant de 4 à 12 atomes de carbone, un radical cycloalkyle ayant de 3 à 12 atomes de carbone, un radical mono ou polycyclique ayant de 5 à 12 atomes de carbone lié au noyau phényle par un carbone tertiaire,

$R_6$ représente un atome d'hydrogène, OH, un radical alkyle inférieur, un radical alkoxy ayant de 1 à 6 atomes de carbone, un radical cycloalkyle ayant de 3 à 12 atomes de carbone, un radical monohydroxyalkyle, un radical polyhydroxyalkyle, un atome de fluor, un atome de chlore, un radical alkényle ayant de 2 à 6 atomes de carbone ou un radical alkényloxy ayant de 2 à 6 atomes de carbone,

$R_5$ et $R_8$ ou $R_6$ et $R_7$ pris ensemble peuvent former avec le cycle aromatique adjacent un cycle à 5 ou 6 chainons éventuellement substitué par des groupes méthyle et/ou éventuellement interrompu par un atome d'oxygène ou de soufre,

X est un radical divalent qui peut être lu de gauche à droite ou inversement choisi dans le groupe constitué par :

(i) - $C(R_{13}R_{14})$ - $C(R_{16}R_{18})$ - W -

(ii) - $C(R_{14}R_{16})$ - W - $C(R_{18}R_{19})$ -

(iii) - $C(R_{13}R_{14})$ - $C(R_{15}R_{16})$ - $C(R_{18}R_{20})$ -

(iv) - $CR_{17}$ = $CR_{21}$ - $C(R_{13}R_{14})$ -

dans lesquels :

W représente un atome d'oxygène, le groupe -$NR_{12}$ ou le groupe S(O)n, n étant 0, 1 ou 2,

$R_{13}$, $R_{15}$ et $R_{20}$ représentent un atome d'hydrogène, le radical - $OR_{11}$, - $OCOR_{11}$, - $NHCOR_{11}$, un radical

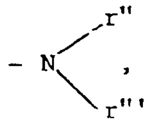

un radical aralkyle, un radical alkyle inférieur, un radical monohydroxyalkyle ou un radical polyhydroxyalkyle,

r'' et r''' identiques ou différents, représentant un atome d'hydrogène, un radical alkyle inférieur, un radical alkényle ayant de 2 à 6 atomes de carbone ou un radical alkynyle ayant de 2 à 6 atomes de carbone,

$R_{14}$, $R_{16}$, $R_{18}$ et $R_{19}$ représentent un atome d'hydrogène, un radical aralkyle, un radical alkyle inférieur, un radical monohydroxyalkyle ou polyhydroxyalkyle,

lorsque X représente (i), $R_{13}$ et $R_{14}$ peuvent former un groupe = N - $OR_{11}$ ou un groupe = N - $OCOR_{11}$

lorsque X représente (iii) ou (iv), $R_{14}$, $R_{16}$ et $R_{18}$ peuvent également représenter le radical - $OR_{11}$ ou le radical - $OCOR_{11}$, ou $R_{13}$, $R_{14}$ et/ou $R_{15}$, $R_{16}$ ou encore $R_{13}$, $R_{14}$ et $R_{18}$, $R_{20}$ pris ensemble peuvent former un groupe oxo, ou encore $R_{13}$, $R_{14}$ ou $R_{15}$, $R_{16}$ pris ensemble peuvent former un groupe = $NOR_{11}$ ou un groupe = N - $OCOR_{11}$,

$R_{12}$ représentant un atome d'hydrogène, un radical alkyle inférieur, un radical aralkyle, un radical alkényle ayant de 2 à 6 atomes de carbone, un radical alkynyle ayant de 2 à 6 atomes de carbone ou un radical fluoroalkyle ayant de 1 à 6 atomes de carbone et de 3 à 7 atomes de fluor,

$R_{17}$ représentant un atome d'hydrogène, un groupe hydroxyle, un radical alkyle inférieur ou un radical alkoxy ayant de 1 à 6 atomes de carbone,

$R_{21}$ représentant un atome d'hydrogène ou un radical alkyle inférieur,

et les sels des composés de formule (V) lorsque $R_1$ représente une fonction acide carboxylique ou lorsque $R_{13}$, $R_{16}$ ou $R_{20}$ représente une fonction amine et les isomères optiques des composés de formule (V), et en particulier :

- l'acide 2-hydroxy-4-[2-hydroxy-2-(5,6,7,8-tétrahydro-5,5,8,8-tétraméthyl-2-naphtyl) éthoxy]benzoïque
- le 2-hydroxy-4- [2-hydroxy-2-(5,6,7,8-tétrahydro-5,5,8,8-tétraméthyl-2-naphtyl) éthoxy]benzoate de méthyle
- l'acide 2-hydroxy-4-[2-hydroxyimino-2-(5,6,7,8-tétrahydro-5,5,8,8-tétraméthyl-2-naphtyl) éthoxy]benzoïque
- l'acide 2-acétyloxy-4-[2-acétyloxy-2-(5,6,7,8-tétrahydro-5,5,8,8-tétraméthyl-2-naphtyl) éthoxy]benzoïque
- l'acide 2-hydroxy-4-[2-acétyloxy-2-(5,6,7,8-tétrahydro-5,5,8,8-tétraméthyl-2-naphtyl) éthoxy]benzoïque
- l'acide 2-acétyloxy-4-[2-hydroxy-2-(5,6,7,8-tétrahydro-5,5,8,8-tétraméthyl-2-naphtyl) éthoxy]benzoïque.

Les composés de formule (V), et en particulier ceux de formules (Va), (Vb), (Vc), (Vd) et (Ve) données ci-après, peuvent être préparés selon les schémas réactionnels suivants :

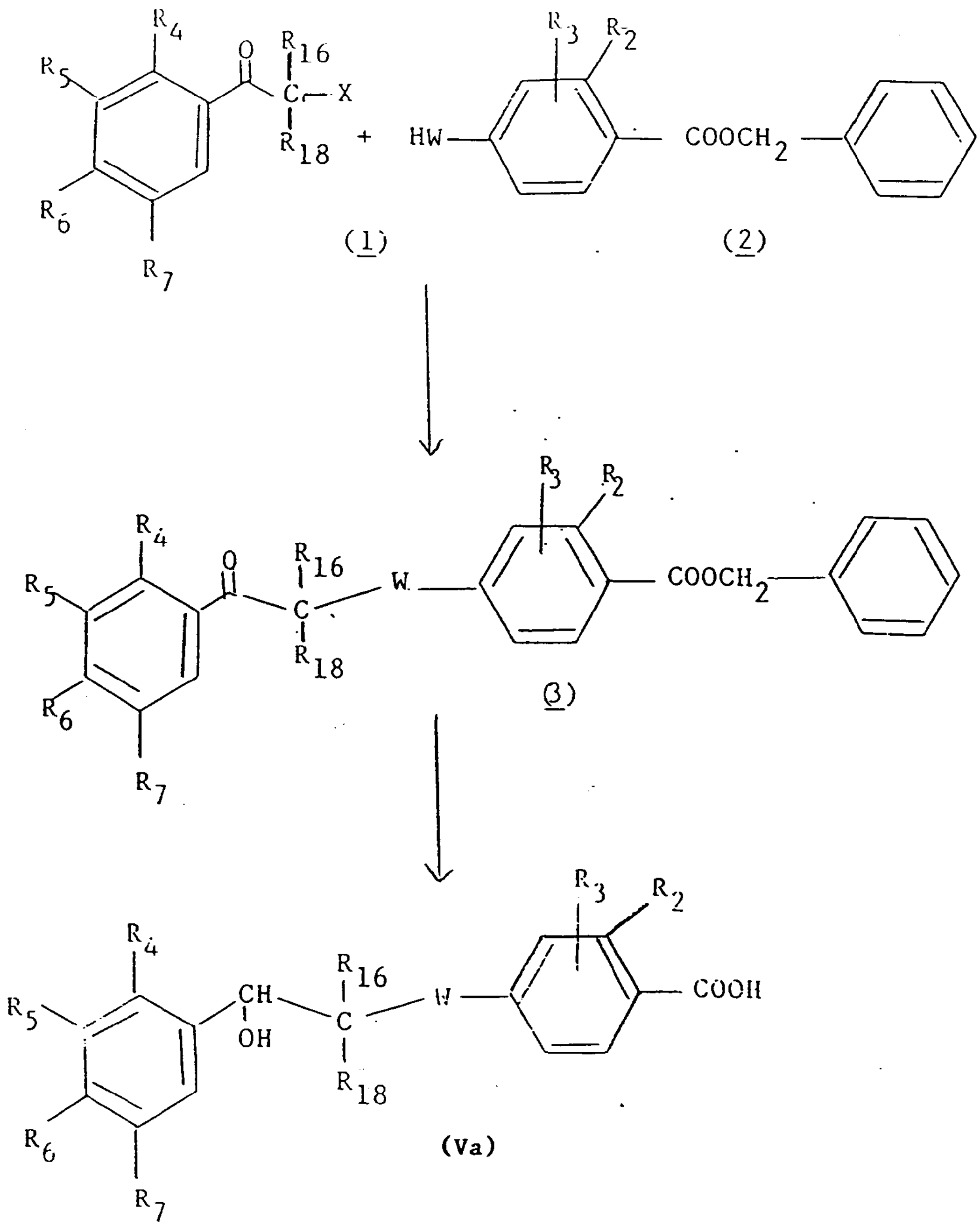

(1) (2)

(3)

(Va)

La première étape de cette préparation consiste à faire réagir en milieu anhydre dans un solvant organique tel que le DMF une α -halocétone (1) avec un parahydroxy, paraamino ou parathiosalicylate de benzyle (2) en présence d'une amine tertiaire (pyridine ou triéthylamine) ou d'un hydrure alcalin (hydrure de sodium) pour obtenir le composé de formule (3).

L'étape principale consiste à hydrogéner le composé de formule (3) en présence d'un catalyseur tel que le palladium sur charbon dans un solvant organique tel que le dioxanne, le méthanol ou le THF.

L'hydrogénation peut s'effectuer à une température entre 20 et 60°C sous une pression d'hydrogène comprise entre 1 bar et 7 bars et permet à la fois d'obtenir l'acide libre et de réduire la fonction cétonique.

Par action de l'hydroxylamine sur le composé (3), on obtient un hydroxymino. La réduction de l'hydroximino permet d'obtenir le composé aminé correspondant.

Les composés de formule générale (V) où X = (i) peuvent aussi être préparés par action d'un chlorure d'acide (5) avec un dérivé aromatique (4) en présence d'un acide Lewis (par exemple $AlCl_3$) dans un solvant chloré tel le dichlorométhane, le dichloroéthane, ou nitré tel le nitrométhane, le nitrobenzène. La cétone (6) ainsi obtenue est réduite en alcool avec un hydrure alcalin tel $NaBH_4$ dans un solvant organique tel que le THF, ou l'éthanol :

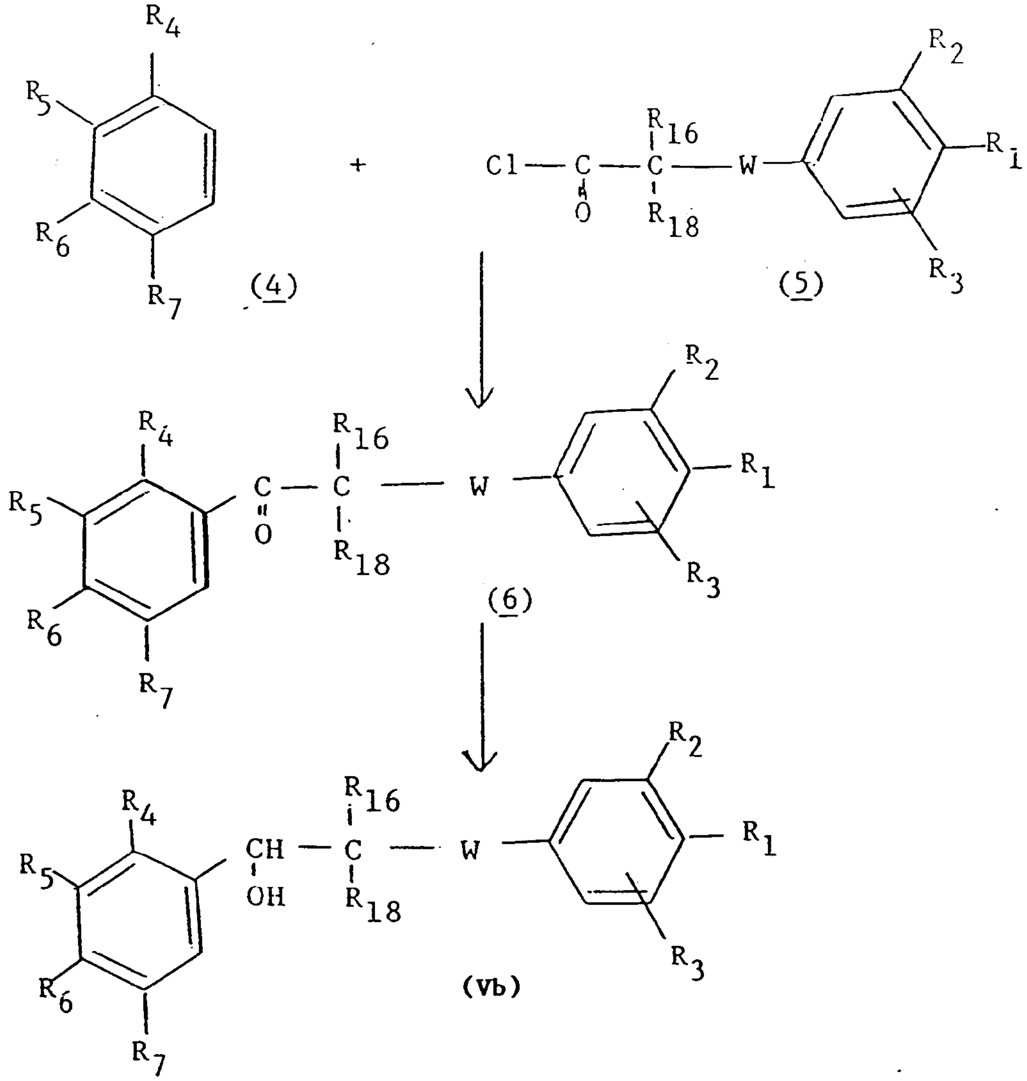

(4) (5) (6) (Vb)

Les composés de formule générale (V) où X = (ii) peuvent être préparés par action d'un bromure de benzyle substitué (7) avec un alcool benzylique ou une amine benzylique ou un mercaptan benzylique substitué(e) (8) en présence d'une amine tertiaire telle que la pyridine ou la triéthylamine dans un solvant organique tel le DMF ou le THF, ou en présence d'un carbonate alcalin, tel le carbonate de potassium dans un solvant tel l'acétone ou la méthyléthylcétone.

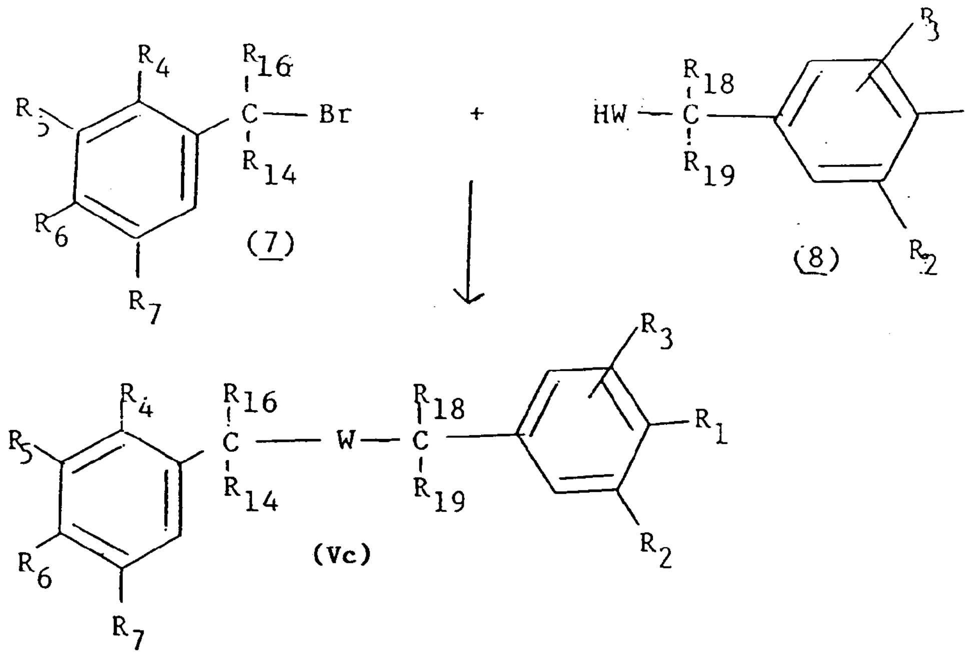

(7) (8)

(Vc)

Les composés de formule générale (V) où X = (iv) peuvent être préparés par action d'une acétophénone substituée (9) avec un benzaldéhyde substitué (10) en présence d'une base telle la soude ou le méthylate de sodium dans un solvant alcoolique (éthanol). La chalcone (11) ainsi obtenue est réduite en alcool allylique (Vd) à l'aide d'un hydrure alcalin tel $NaBH_4$ dans un solvant alcoolique en présence d'un catalyseur ($CeCl_3$).

Par hydrogénation du composé (Vd) en présence d'un catalyseur tel le palladium sur charbon dans un solvant tel le dioxanne ou le méthanol on obtient des composés (Ve) de formule générale (V) où X = (iii).

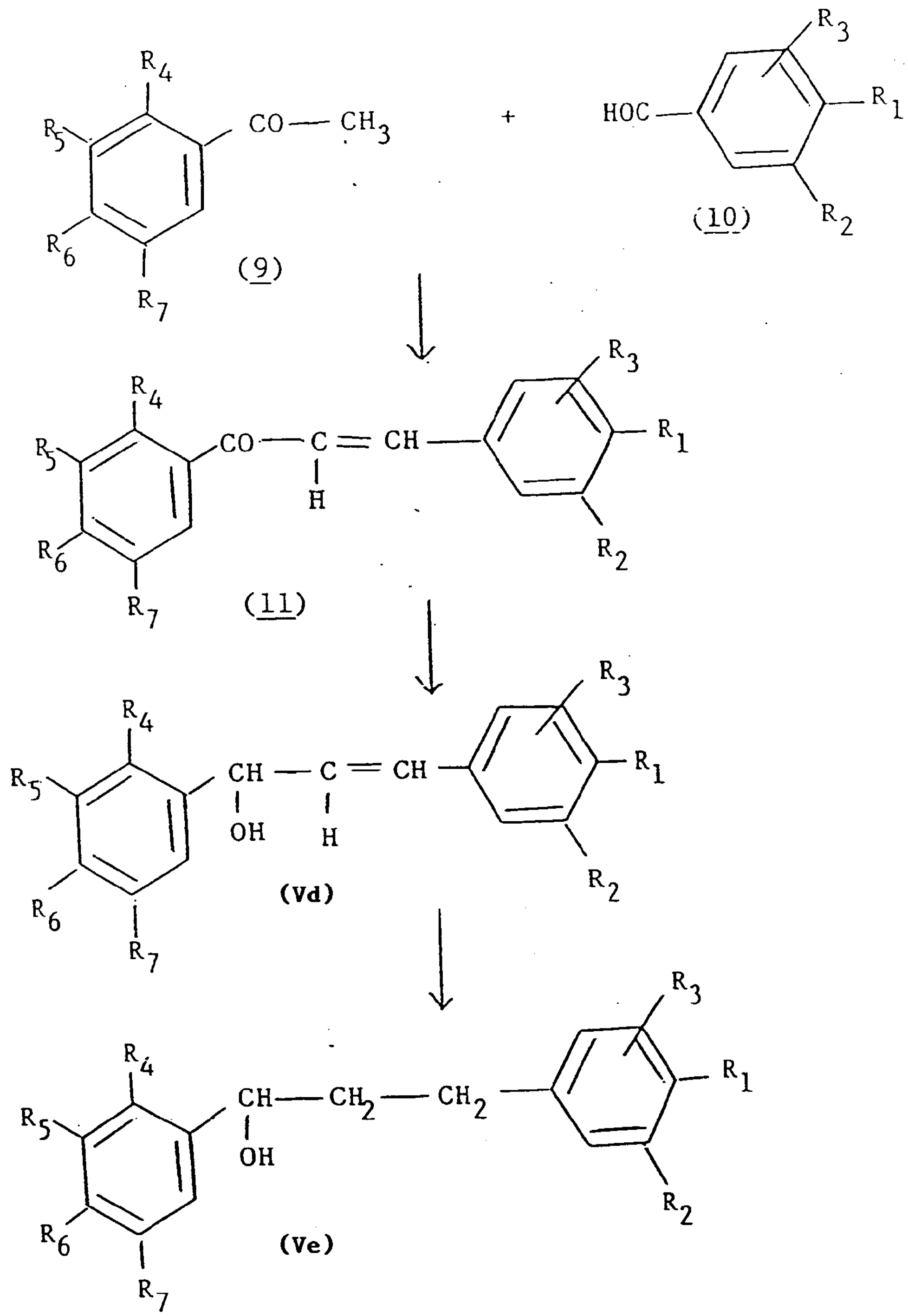

(9) (10)

(11)

(Vd)

(Ve)

Par réaction de type Mitsunobu à partir des alcools (Vb), (Vd) ou (Ve) on obtient le dérivé azido que l'on peut transformer en dérivé amino.

Par réaction d'un anhydride ou d'un chlorure d'acide sur le dérivé amino, on obtient l'amide correspondant.

Dans le cas où le radical X est lu de manière inversée par rapport aux schémas précédents aboutissant aux composés (Va), (Vb), (Vd) ou (Ve), les composés sont obtenus par les réactions décrites dans ces schémas en utilisant des produits de départ ayant des substituants appropriés.

Lorsque X représente un radical di ou trihydroxylé, les composés de formule (V) sont obtenus par époxydation des composés éthyléniques correspondants et ouverture de la fonction époxy en milieu alcalin ou en présence d'un hydrure.

- les rétinoïdes aromatiques dérivés d'imine décrits dans la demande de brevet FR 91-05883 de formule générale (VI) :

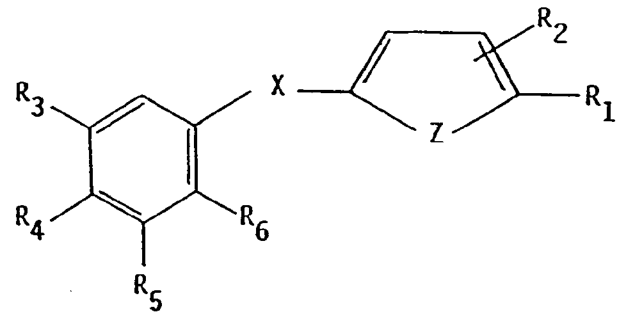

(VI)

dans laquelle :

$R_1$ représente un atome d'hydrogène, le groupe OH, le radical $-CH_3$, le radical $-CH_2OH$, le radical $-COR_7$, le radical $-CH(OH)CH_3$, le radical $-CH_2OCOR_8$, le radical $-SO_2R_9$, le radical $SOR_9$ ou le radical $-SR_9$,

$R_7$ représentant un atome d'hydrogène, le groupe OH, le radical $-OR_{10}$, le radical :

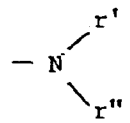

un radical alkyle inférieur, un radical monohydroxyalkyle, un radical polyhydroxyalkyle ou le reste d'un sucre,

$R_8$ représentant un radical alkyle linéaire ou ramifié, ayant de 1 à 20 atomes de carbone, un radical alkényle ayant de 2 à 20 atomes de carbone ou le reste d'un sucre,

$R_9$ représentant le groupe OH, un radical alkyle inférieur ou le radical

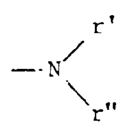

$R_{10}$ représentant un radical alkyle ayant de 1 à 20 atomes de carbone ou un radical alkényle ayant de 2 à 20 atomes de carbone,

r′ et r″, identiques ou différents, représentent un atome d'hydrogène, un radical alkyle inférieur, un radical aryle, un radical aralkyle, un reste d'aminoacide, un reste de sucre, un reste de sucre aminé ou un hétérocyle ou r′ et r″ pris ensemble forment un hétérocycle,

$R_2$ et $R_6$ représentent un atome d'hydrogène, le groupe OH, un radical alkyle inférieur, un radical alkoxy ayant de 1 à 6 atomes de carbone, un atome de fluor, un atome de chlore ou le groupe $CF_3$,

$R_3$ et $R_5$ représentent un radical alkyle $\alpha,\alpha'$-disubstitué ayant de 4 à 12 atomes de carbone ou un radical cycloalkyle mono ou polycyclique ayant de 5 à 12 atomes de carbone dont le carbone de liaison est trisubstitué,

$R_4$ représente un atome d'hydrogène, le groupe OH, un radical alkoxy ayant de 1 à 6 atomes de carbone, un radical alkyle $\alpha,\alpha'$-disubstitué ayant de 4 à 12 atomes de carbone,

$R_3$ et $R_4$ ou $R_4$ et $R_5$ pris ensemble peuvent former, avec le noyau benzénique adjacent, un cycle à 5 ou 6 atomes de carbone substitués par 2 à 6 groupes méthyles,

Z représente un atome d'oxygène ou de soufre, le radical divalent $-CH=CR_{11}-$ ou le radical divalent $-N=CR_{12}-$

$R_{11}$ représentant un atome d'hydrogène, le groupe OH ou un radical alkyle inférieur,

$R_{12}$ représentant un atome d'hydrogène ou un radical alkyle inférieur,

X est choisi parmi les radicaux :

(i) $- CR_{13}= N -$

(ii) $- N = CR_{13}-$

(iii)

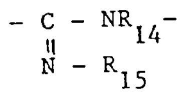

(iv)

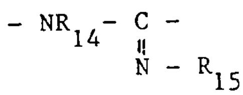

$R_{13}$ représentant le radical $R_{16}$, le radical $OR_{16}$, le radical $-SR_{16}$ ou le radical :

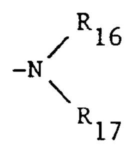

$R_{16}$ et $R_{17}$ représentant un atome d'hydrogène, un radical alkyle inférieur, un radical fluoroalkyle inférieur, un radical alkényle ayant de 2 à 6 atomes de carbone, un radical alkynyle ayant de 2 à 6 atomes de carbone, un radical aryle ou un radical aralkyle,

$R_{14}$ représentant un radical alkyle inférieur,

$R_{15}$ représentant un radical alkyle inférieur ou un radical fluoroalkyle inférieur,

et en particulier :

- l'acide 4-(N-méthyl-5,6,7,8-tétrahydro-5,5,8,8-tétraméthyl-2-naphtylcarboxamidino benzoïque
- l'acide 4-(5,6,7,8-tétrahydro-5,5,8,8-tétraméthyl-2-naphtylcarboxamidino) benzoïque

Lorsque X représente une liaison imine avec X = (i) les composés (VI) sont obtenus par réaction d'un benzaldéhyde substitué avec un para-amino benzoate d'allyle éventuellement substitué dans un solvant anhydre tel le chlorure de méthylène en présence d'un déshydratant par exemple l'alumine basique.

Lorsque X représente une liaison imine avec X = (ii) les composés (VI) sont obtenus par réaction d'une aniline substituée sur un aldéhyde aromatique substitué par une fonction acide qui est protégée sous forme d'ester allylique dans les mêmes conditions que précédemment.

Lorsque X représente une liaison imidate, thioimidate ou amidine avec X = (i) les composés (VI) sont préparés d'après le schéma réactionnel suivant :

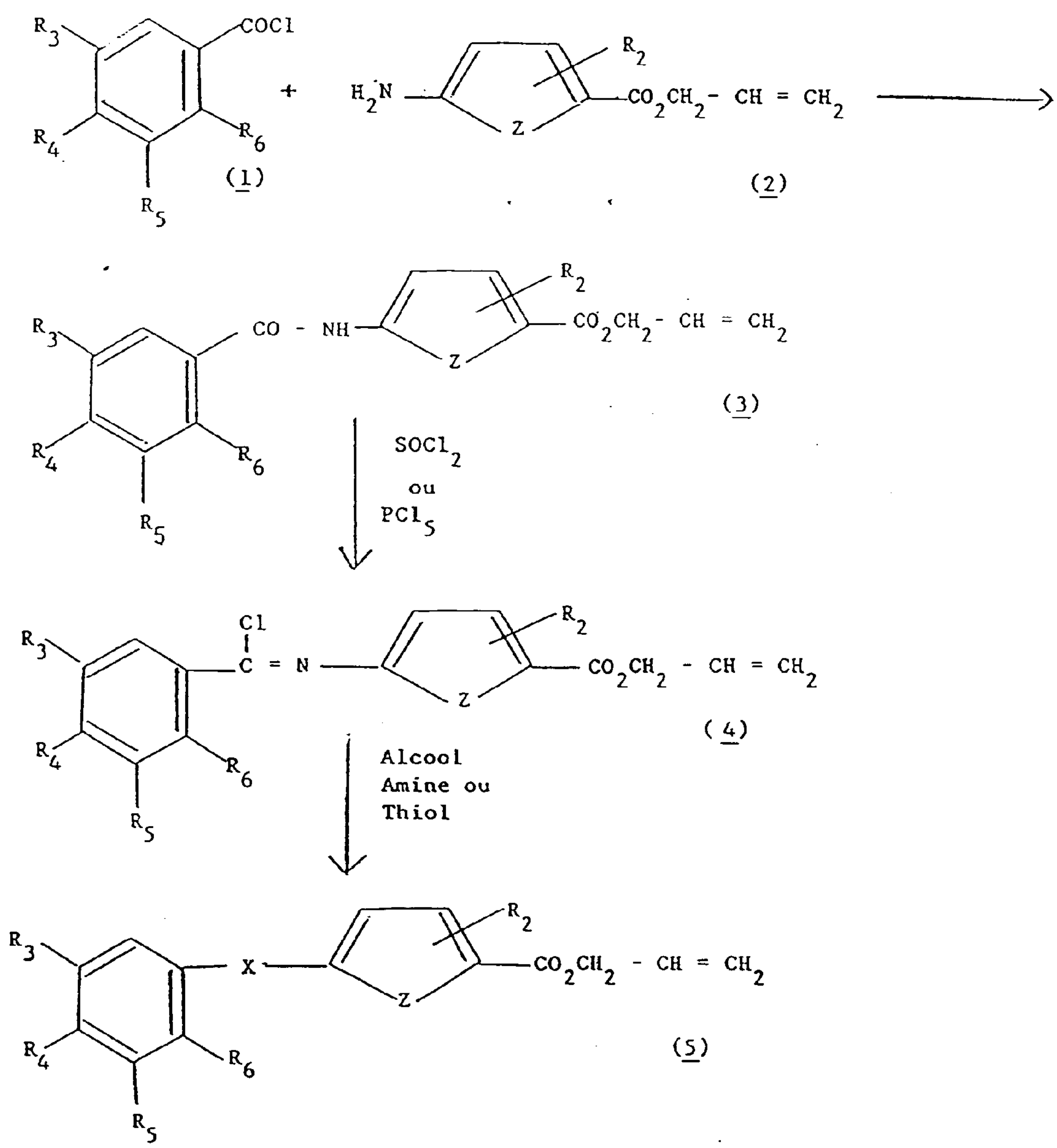


(1) (2) (3) (4) (5)

La première étape consiste à faire réagir en milieu anhydre, dans un solvant organique tel que le tétrahydrofuranne ou le chlorure de méthylène contenant une amine tertiaire (pyridine ou triéthylamine) une forme activée d'un acide benzoïque substitué, par exemple un chlorure d'acide (1) ou un anhydride mixte sur un para-amino benzoate d'allyle éventuellement substitué (2) . La réaction est conduite à température ambiante et sous agitation.

L'amide (3) ainsi obtenu est converti en iminochlorure (4) par action du chlorure de thionyle, du pentachlorure de phosphore ou du phosgène.

Par réaction du composé (4) avec une amine, un alcool ou un thiol en présence d'une amine tertiaire et d'un hydrure alcalin dans un solvant organique tel que le tétrahydrofuranne ou le chlorure de méthylène, on obtient le composé de formule (5).

Lorsque X représente une liaison imidate, thioimidate, ou amidine avec X = (ii) la préparation est effectuée de la même manière que précédemment en partant des composés (6) et (7) suivants :

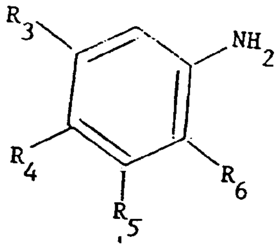

(6)

\+

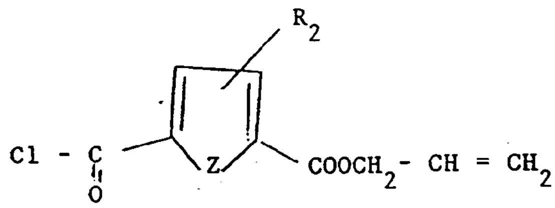

(7)

Le passage de l'ester à l'acide libre peut être effectué dans les 4 cas ci-dessus au moyen d'un catalyseur, tel que certains complexes de métaux de transition par exemple le tetrakis triphényl phosphine palladium (0) en présence d'une amine secondaire ou du sel de sodium du malonate de diéthyle.

Lorsque X représente une liaison amidine correspondant aux formules (iii) et (iv) la synthèse est réalisée selon le procédé classique de PINNER en condensant une aniline substituée sur un nitrile aromatique.

Les stérols utilisables dans la composition de l'invention sont ceux qui sont capables d'inhiber la biosynthèse du cholestérol. On entend ici par "stérol" des composés ayant le squelette hydrocarboné de cholestérol ou du lanostérol, et portant en outre au moins un substituant alkylé, oxygéné et/ou azoté, et/ou au moins une insaturation.

On sait que, chez l'homme par exemple, le cholestérol est synthétisé principalement dans le foie et l'intestin, mais aussi dans d'autres organes, dont la peau. Le point de départ est l'acétyl coenzyme A (en abrégé acétyl CoA) qui donne, par une réaction de condensation, l'acétoacétyl CoA, qui, par réaction avec une autre molécule d'acétyl CoA, fournit la bêta-hydroxy bêta-méthyl-glutaryl CoA (en abrégé HMG CoA). Ces réactions sont catalysées par la HMG CoA synthétase. Le HMG CoA est réduit en acide mévalonique par la HMG CoA réductase. L'acide mévalonique, par réaction avec 3 ATP, fixe trois phosphoryles et le composé obtenu se transforme par décarboxylation en isopentényl pyrophosphate. Ce dernier composé s'isomérise, sous l'action d'une isomérase, en diméthylallyl pyrophosphate. Par réaction de deux molécules d'isopentényl pyrophosphate et d'une molécule de diméthylallyl pyrophosphate, on obtient un trimère, le farnésil pyrophosphate, en passant par l'intermédiaire d'un dimère (le géranyl pyrophosphate). Deux molécules du trimère se condensent pour former un hexamère, le squalène. Par une réaction de cyclisation complexe, dans laquelle intervient notamment la 2,3-oxydosqualène cyclase, le squalène se transforme en 24,25-dihydrolanostérol puis en lanostérol. Le lanostérol est ensuite transformé en 14-desméthyl lanostérol qui, par la perte des deux méthyle en position 4, donne le zymostérol qui s'isomérise en cholesta-7,24-diènol, puis en desmostérol. Par réduction de la double liaison 24-25, on obtient finalement le cholestérol.

Les stérols utilisables dans la composition de l'invention sont ceux qui sont capables d'inhiber la biosynthèse du cholestérol à un stade quelconque. Il peut s'agir notamment des inhibiteurs de l'HMG CoA réductase, des inhibiteurs de la 2,3-oxydosqualène cyclase, des inhibiteurs du métabolisme du 24,25-dihydrolanostérol, des inhibiteurs de la conversion du lanostérol en desmostérol ou des inhibiteurs de la conversion du desmostérol en cholestérol.

Parmi les inhibiteurs de l'HMG CoA réductase, on citera notamment : les stérols portant sur la structure cholestérol ou sur une structure dérivée, au moins un substituant oxygéné autre que l'hydroxyle en position 3. Le substituant oxygéné peut être par exemple un hydroxyle, un oxo, un époxy, un peroxyde ou un carbonyle. Ce substituant peut être par exemple en position 4, 7, 14, 15, 20, 22, 24, 25 ou 26. On peut citer à titre d'exemple le 25-hydroxycholestérol.

Parmi les inhibiteurs de l'HMG CoA réductase on peut également citer le 6-nitrocholestérol.

Parmi les inhibiteurs de la 2,3-oxydosqualène cyclase, on citera par exemple le 2-aza-2,3-dihydrosqualène ou ses dérivés, et le N-[(1,5,9)-triméthyldécyl]-4 $\alpha$,10-diméthyl-8-aza-trans-décal-3 $\beta$-ol.

Parmi les inhibiteurs du métabolisme du 24,25-dihydrolanostérol, on peut citer certains dérivés oxygénés et insaturés en position 8-9 du lanostérol et en particulier :

- le 7-oxolanost-8-èn-3 $\beta$-ol,
- le 3 $\beta$-acétoxylanost-8-èn-7-one,
- le 7-oxolanost-5,8,11-trièn-3 $\beta$-ol.

Parmi les inhibiteurs de la conversion du desmostérol en cholestérol, on peut citer notamment le 20,25-diazacholestérol.

Parmi les inhibiteurs de la biosynthèse du cholestérol, à un stade postérieur à la biosynthèse du lanostérol, selon la voie métabolique lanostérol → cholestérol, on peut citer notamment le 22-oximino cholestérol.

Dans la composition de l'invention, le rétinoïde est généralement présent en proportions pouvant aller de 0,0001 à 50 % en poids, par rapport au poids total de la composition, et le rapport de la concentration molaire du stérol à la concentration molaire du rétinoïde peut varier de 0,1 à 10.

La composition de l'invention contient, outre les ingrédients actifs mentionnés ci-dessus, un véhicule pharmaceutique compatible avec une administration par voie topique (peau et muqueuses), oculaire ou systémique.

Les deux constituants de l'association synergétique peuvent en outre être administrés séparément, par la même voie ou par des voies différentes, et l'invention a également pour objet une composition en deux parties comprenant, réunis dans un emballage approprié, d'une part au moins un rétinoïde, tel que défini ci-dessus, dans un conteneur approprié, et, dans un second conteneur, au moins un stérol inhibiteur de la biosynthèse du cholestérol. Cette composition en deux parties contient en outre un mode d'emploi préconisant l'administration simultanée ou séquentielle des deux principes actifs.

Pour l'application par voie topique, les compositions pharmaceutiques ou cosmétiques de l'invention comprennent les véhicules et ingrédients nécessaires pour permettre de présenter la composition par exemple sous la forme d'onguents, de crèmes, de laits, de pommades, de poudres, de tampons imbibés, de solutions, de gels, de sprays, de shampooings, de lotions lavantes, ou encore de suspensions, de microsphères ou nanosphères, de vésicules lipidiques ou polymériques, ou de patches polymériques.

Pour l'administration par voie oculaire, la composition de l'invention se présente sous la forme de collyres.

Pour l'administration par voie buccale, la composition de l'invention se présente sous forme de solution, suspension, gel, pâte, vésicules lipidiques ou polymériques.

Dans les compositions pour la voie topique oculaire ou buccale, le rétinoïde est présent, par exemple, en proportions pouvant aller de 0,0001 à 1 % en poids par rapport au poids total de la composition. On applique généralement la composition 1 à 2 fois par jour sur la zone à traiter.

Pour l'administration par voie systémique, les compositions pharmaceutiques de l'invention peuvent se présenter sous la forme de comprimés, de gelules, de dragées, de sirops, de poudres à diluer, de granulés, de microsphères, de nanosphères, de vésicules lipidiques ou polymériques, d'émulsions, de suspensions ou de solutions pour l'administration par voie orale, par perfusion ou par injection.

Dans les compositions administrées par la voie orale, le rétinoïde est généralement présent en porportions pouvant aller de 0,01 à 50 % en poids par rapport au poids total de la composition.

Dans les compositions administrées par perfusion ou par injection, le rétinoïde est généralement présent en proportions pouvant aller de 0,0001 à 1 % en poids par rapport au poids total de la composition.

L'invention a également pour objet l'utilisation, en association, d'au moins un rétinoïde et d'au moins un stérol, dans la préparation d'une composition pharmaceutique ou cosmétique destinée notamment à traiter ou corriger les troubles de la kératinisation épidermique, tout autre trouble ou tout autre défaut ou excès fonctionnel de la prolifération épidermique ou épithéliale, et/ou les troubles de la fonction sébacée. La composition ainsi préparée peut servir à traiter les troubles mentionnés ci-dessus, ayant ou non une composante inflammatoire et/ou immunoallergique, y compris les maladies de dégénérescence du tissu conjonctif et les tumeurs bénignes ou malignes, à lutter contre le vieillissement cutané, à favoriser la cicatrisation. ou à améliorer l'aspect de la peau des personnes présentant des troubles de la kératinisation ou souffrant de séborrhée.

En particulier, l'association décrite dans la présente invention est destinée :

- au traitement des affections dermatologiques liées à un désordre de la kératinisation portant sur la différenciation et sur la prolifération, notamment les acnés vulgaires, comédoniennes ou polymorphes, les acnés nodulo-kystiques, conglobata, les acnés séniles, les acnés secondaires telles que l'acné solaire, médicamenteuse, professionnelle ;
- au traitement d'autres types de troubles de la kératinisation, notamment les ichtyoses, les états ichtyosiformes, la maladie de Darier, les kératodermies palmoplantaires, les leucoplasies et les états leucoplasiformes ainsi que le lichen ;
- au traitement d'autres affections dermatologiques liées à un trouble de la kératinisation avec une composante inflammatoire et/ou immunoallergique et notamment, toutes les formes de psoriasis qu'il soit cutané, muqueux ou unguéal, et même le rhumatisme psoriatique, ou encore l'atopie cutanée telle que l'eczéma ;
- au traitement de toutes les proliférations cutanées, bénignes ou malignes, qu'elles soient d'origine virale telles que les verrues vulgaires, les verrues planes et l'épidermodysplasie verruciforme, ou qu'elles soient induites par les ultraviolets comme par exemple dans le cas des épithélioma baso- et spino-cellulaires, dans le traitement de situations cancéreuses ou précancéreuses, en particulier au niveau cutané ;
- au traitement d'autres désordres dermatologiques tels que les dermatoses bulleuses et les maladies du collagène ;

- à lutter contre le vieillissement de la peau, qu'il soit photo-induit ou chronologique ou, à réduire les pigmentations et kératoses actiniques ;
- à prévenir ou guérir les stigmates de l'atrophie épidermique et/ou dermique induite par les corticostéroïdes locaux ou systémiques, ou toute autre forme d'atrophie cutanée ;
- à favoriser la cicatrisation ;
- au traitement de certains troubles ophtalmologiques, notamment les cornéopathies ;
- à lutter contre les troubles de la fonction sébacée tels que l'hyperséborrhée de l'acné ou la séborrhée simple ;
- pour la réparation des altérations des fibres collagéniques et élastiques dermiques, telles que les vergetures de croissance, les vergetures post-corticoïdes et les vergetures au décours d'une grossesse. Dans ce dernier cas, le traitement devra être effectué après la grossesse ;
- à lutter conter les affections dégénératives ou destructives ou inflammatoires du parodonte et particulièrement les gingivites et les parodontoclasies.

Les compositions de l'invention trouvent également une application dans le domaine cosmétique, en particulier dans l'hygiène corporelle, et aussi capillaire (action contre la séborrhée).

Les exemples suivants illustrent l'invention

EXEMPLES DE FORMULATIONS

EXEMPLES 1 et 2 : Lotions

On a préparé les lotions suivantes :

EXEMPLE 1 :

| | |
|---|---|
| Polyéthylène glycol 400................................ | 49,850 g |
| Ethanol à 95 %......................................... | 50,000 g |
| Acide 2-(5,6,7,8-tétrahydro-5,5,8,8-tétraméthyl 2-naphtyl) -5 benzimidazole carboxylique.......................... | 0,025 g |
| 25-hydroxy cholestérol................................. | 0,075 g |
| Butylhydroxytoluène.................................... | 0,050 g |

EXEMPLE 2 :

| | |
|---|---|
| Polyéthylène | 59,89 g |
| Ethanol à 95 % | 30 g |
| Isopropanol | 10 g |
| Butylhydroxytoluène | 0,05 g |
| Acide rétinoïque | 0,01 g |
| 25-hydroxy cholestérol | 0,05 g |

On applique ces lotions sur la peau. deux fois par jour.

EXEMPLE 3 et 4 : Gels

On a préparé les gels suivants :

EXEMPLE 3 :

| | |
|---|---|
| Hydroxypropylcellulose | 2,50 g |
| Ethanol à 95 % | 97,41 g |
| Butylhydroxytoluène | 0,05 g |
| Acide rétinoïque | 0,01 g |
| 25-hydroxy cholestérol | 0,03 g |

EXEMPLE 4 :

| | |
|---|---|
| Myristate d'isopropyle | 89,90 g |
| Silice colloïdale | 10 g |
| α-tocophérol | 0,025 g |
| Acide rétinoïque | 0,025 g |
| 25-hydroxy cholestérol | 0,05 g |

Ces gels sont appliqués sur les zones atteintes de la peau deux fois par jour.

EXEMPLE 5 : Emulsion E/H (Eau-dans-l'huile)

On a préparé l'émulsion suivante en solubilisant à chaud (60°C-70°C) sous atmosphère inerte et sous agitation mécanique, d'une part le parahydroxybenzoate de méthyle dans l'eau distillée et d'autre part le parahydroxybenzoate de propyle dans l'eucérine. En maintenant la température et en restant sous atmosphère inerte, la partie aqueuse est ensuite émulsionnée lentement dans l'eucérine sous agitation mécanique. On laisse la température redescendre à 40°C avant d'incorporer à l'abri de la lumière et sous agitation l'α-tocophérol, le 25-hydroxy cholestérol, puis l'acide rétinoïque.

| | |
|---|---|
| Eucerine anhydre | 40 g |
| Parahydroxybenzoate de méthyle | 0,075 g |
| Parahydroxybenzoate de propyle | 0,075 g |
| α-tocophérol | 0,050 g |
| Eau distillée | 59,70 g |
| Acide rétinoïque | 0,025 g |
| 25-hydroxy cholestérol | 0,075 g |

L'eucérine anhydre est un mélange d'alcools de lanoline émulsifs, de cires et d'huiles raffinées à base d'hydrocarbures, vendu par la Société BDF.

EXEMPLES 6 et 7 : EMULSIONS H/E (Huile-dans-l'eau)

On a préparé les émulsions suivantes en solubilisant à chaud (70°C) sous atmosphère inerte, d'une part, les conservateurs liposolubles dans le mélange fondu de corps gras et d'émulsionnants et d'autre part, le parahydroxy benzoate de méthyle dans l'eau distillée. En maintenant la température, et en restant sous atmosphère inerte, la partie lipidique est ensuite émulsionnée dans la partie aqueuse sous agitation mécanique. On laisse la température revenir à 40°C avant d'incorporer à l'abri de la lumière et sous agitation le 25-hydroxycholestérol, puis l'acide rétinoïque.

EXEMPLE 6

| | |
|---|---|
| Lauryl sulfate de sodium | 1,00 g |
| 1-2 propanediol | 10,00 g |
| 1-Hexadécanol | 18,00 g |
| Parahydroxybenzoate de propyle | 0,075 g |
| Parahydroxybenzoate de méthyle | 0,075 g |
| Butylhydroxytoluène | 0,050 g |
| Eau distillée | 70,76 g |
| Acide rétinoïque | 0,010 g |
| 25-hydroxy cholestérol | 0,030 g |

EXEMPLE 7 :

| | |
|---|---|
| Stéarate de glycéryle | 15,00 g |
| Monostéarate de sorbitan | 4,00 g |
| Monostéarate de sorbitan polyoxyéthyléné à 20 moles d'oxyde d'éthylène | 1,00 g |
| 2-octyldodécanol | 10,00 g |
| Parahydroxybenzoate de méthyle | 0,15 g |
| Eau distillée | 69,805 g |
| Butylhydroxytoluène | 0,01 g |
| Acide rétinoïque | 0,01 g |
| 25-hydroxy cholestérol | 0,025 g |

Les émulsions des exemples 5 à 7, qui constituent des crèmes, peuvent être appliquées sur les zones atteintes de la peau, deux fois par jour.

EXEMPLE 8 :

Gel-crème

| | | |
|---|---|---|
| Rétinol palmitate | | 0,050 g |
| 25-hydroxy cholestérol | | 0,050 g |
| Lécithine de soja hydrogénée | | 5,000 g |
| α-tocophérol | | 0,005 g |
| Parahydroxybenzoate de méthyle | | 0,100 g |
| Parahydroxybenzoate de propyle | | 0,050 g |
| BHT | | 0,050 g |
| Polymère carboxyvinylique (Carbopol 940) | | 0,500 g |
| NaOH 5 % | qs | pH 7,0 |
| Eau stérile | qs | 100,000 g |

EXEMPLE 9 :

Gel gingival

| | |
|---|---|
| Composé 1 * .......................................... | 0,025 g |
| 6-nitrocholestérol .................................. | 0,075 g |
| Hydroxypropyl cellulose ............................. | 2,500 g |
| Parahydroxybenzoate de méthyle ...................... | 0,150 g |
| Parahydroxybenzoate de propyle ...................... | 0,050 g |
| Propylène glycol .................................... | 5,000 g |
| Eau stérile qs ...................... | 100,000 g |

* Composé 1 : Acide 2-(5,6,7,8-tétrahydro-5,5,8,8-tétraméthyl-2-naphtyl) -5-benzimidazole carboxylique.

ACTIVITE DES COMPOSES SUR LA FORMATION DES ENVELOPPES CORNEES (EC) DANS LES CULTURES DE KERATINOCYTES HUMAINS TRANSFORMES PAR SV-40

L'activité de l'association entre rétinoïde et stérol sur la formation des enveloppes cornées a été évaluée selon la méthode décrite par S. Michel et coll. dans Models Dermatol, Maibach et Lowe Eds, Karger, Basel Vol. 4, p.40-44 (1989).

EXEMPLE I : Acide Rétinoïque et 25-hydroxy cholestérol

| COMPOSES | DOSE(S) | INHIBITION DE LA FORMATION DES EC |
|---|---|---|
| Acide Rétinoïque | 0,01 µM<br>1,00 µM | 0% - 10%<br>80% - 100% |
| 25-OH Cholestérol | 0,026 µM | 0% - 10% |
| Acide Rétinoïque<br>+<br>25-OH Cholestérol | 0,01 µM<br>+<br>0,026 µM | 80% - 100% |

EXEMPLE II : Composé 1* et 25-hydroxy cholestérol

| COMPOSES | DOSE(S) | INHIBITION DE LA FORMATION DES EC |
|---|---|---|
| Composé 1 | 0,50 µM | 10% |
| 25-OH Cholestérol | 1,28 µM | 55% |
| Composé 1 + 25-OH Cholestérol | 0,50 µM + 1,28 µM | 100% |
| Composé 1 + 25-OH Cholestérol | 0,25 µM + 0,64 µM | 100% |

* Composé 1 : Acide 2-(5,6,7,8-tétrahydro-5,5,8,8-tétraméthyl 2-naphtyl)-5-benzimidazole carboxylique.

## Revendications

### Revendications pour les Etats contractants suivants : AT, BE, CH, DE, DK, FR, GB, IT, LI, LU, NL, SE

1. Composition pharmaceutique ou cosmétique caractérisée par le fait qu'elle comprend en association :
   - au moins un rétinoïde capable d'inhiber l'expression de la transglutaminase membranaire,
   - et au moins un stérol inhibiteur de la biosynthèse du cholestérol.

2. Composition selon la revendication 1, caractérisée par le fait que ledit rétinoïde est choisi parmi :
   - Acide rétinoïque all-trans ou 13-cis,
   - Acide (all-E)-9-(4-méthoxy-2,3,6-triméthylphényl)-3,7-diméthyl-2,4,6,8-nonatétraénoïque,
   - Acide (E,E,E)-7-(2,3-dihydro-1,1,3,3-tétraméthyl-1H-indèn-5-yl)-3,7-diméthyl-2,4,6-octatriènoïque,
   - Acide (E,E,E)-7-(5,6,7,8-tétrahydro-5,5,8,8-tétraméthyl-2-naphthalényl-3,7-diméthyl-2,4,6-octatriènoïque,
   - Acide (E)-4-[(2,3-dihydro-1,1,3,3-tétraméthyl-1H-indèn-5-yl)1-propènyl] benzoïque,
   - Acide (E)-4-[2-(5,6,7,8-tétrahydro-5,5,8,8-tétraméthyl-2-naphtalènyl)-1-propènyl] benzoïque,
   - Acide 2-(5,6,7,8-tétrahydro-5,5,8,8-tétraméthyl-2-naphtyl)-6-naphtalène carboxylique,
   - Acide (E)-4-[2-(5,6,7,8-tétrahydro-5,5,8,8-tétraméthyl-2-naphtalènyl)-1-propènyl] benzène sulfonique
   - Acide (E,E)-4-[2-méthyl-4-(2,6,6-triméthyl-1-cyclohexèn-1-yl)-1,3-butadiènyl]benzoïque,
   - Acide (E,E)-4-[4-méthyl-6-(2,6,6-triméthyl-1-cyclohexèn-1-yl)-1,3,5-hexatriènyl] benzoïque,
   - Acide (E)-6-[2-(2,6,6-triméthyl-1-cyclohexèn-1-yl)-éthènyl]-2-naphthalènecarboxylique,
   - Acide (E,E)-5-[2-méthyl-4-(2,6,6-triméthyl-1-cyclohexèn-1-yl)-1,3-butadiènyl]-2-thiophène carboxylique,
   - Acide (E)-4-[2-(5,6,7,8-tétrahydro-8,8-diméthyl-2-naphtalènyl)-1-propènyl] benzoïque,
   - Acide 4-[(5,6,7,8-tétrahydro-5,5,8,8-tétraméthyl-2-naphtalènyl) éthynyl]benzoïque,
   - Acide (E)-4-[2-(5,6,7,8-tétrahydro-3-méthyl-5,5,8,8-tétraméthyl-2-naphtalènyl)-1-propènyl]benzoïque,
   - Acide 4-(5,6,7,8-tétrahydro-5,5,8,8-tétraméthyl-2-naphtalènylcarbamoyl) benzoïque,
   - Acide 4-(5,6,7,8-tétrahydro-5,5,8,8-tétraméthyl-2-naphtamido) benzoïque,
   - Acide (E)-4-[3-(3,5 di-tert-butylphényl)-3-oxo-1-propènyl] benzoïque,

- Acide 6-[(5,6,7,8-tétrahydro-5,5,8,8-tétraméthyl-2-naphtalènyl) éthynyl]3-pyridine carboxylique,
- Acide 2-(5,6,7,8-tétrahydro-5,5,8,8-tétraméthyl-2-naphtyl)-6-benzo(b) thiophène carboxylique,
- Acide 2-(5,6,7,8-tétrahydro-5,5,8,8-tétraméthyl-2-naphtyl)-6-benzo(b) furanecarboxylique,
- Acide 2-(5,6,7,8-tétrahydro-5,5,8,8-tétraméthyl-2-naphtyl)-6-indole carboxylique,
- Acide 2-[3-(1-adamantyl)-4-méthoxyphényl]-5 benzimidazole carboxylique,
- Acide 2-(5,6,7,8-tétrahydro-5,5,8,8-tétraméthyl-2-naphtyl)-5-benzimidazole carboxylique,
- Acide 4-(5,6,7,8-tétrahydro-5,5,8,8-tétraméthyl-2-anthracényl) benzoïque,
- Acide 6-[3-(1-adamantyl)-4-méthoxyphényl]-2-naphtoïque,
- Acide 4-[3-(1-adamantyl)-4-méthoxybenzamido]benzoïque,
- Acide 4-[3-(1-adamantyl)-4-méthoxy benzoylthio]benzoïque,
- Acide 4-[3-(1-adamantyl)-4-méthoxy benzoyloxy]benzoïque,
- Acide 2- (5,6,7,8-tétrahydro-5,5,8,8-tétraméthyl-2-naphtyl) 6-carbonyl naphtalène carboxylique,
- Acide p-(5,6,7,8-tétrahydro-5,5,8,8-tétraméthyl-2-1H-benz[f]indolyl) benzoïque,
- Acide trans-(5,5,8,8-tétraméthyl-5,6,7,8 tétrahydro-2-naphtyl)-4-carbonyl-α-méthyl cinnamique,
- la vitamine A ou rétinol, ainsi que ses esters, tels que l'acétate, le propionate ou le palmitate de rétinol
- l'aldéhyde de vitamine A ou rétinal
- l'acide 4-[3-(1-adamantyl)-4-méthoxybenzoyloxy] -2-fluorobenzoïque
- l'acide 4-[3-(1-adamantyl)-4-méthoxybenzoyloxy]-2-methylbenzoïque
- l'acide 4-[3-(1-adamantyl)-4-méthoxybenzoyloxy]-2-hydroxybenzoïque
- l'acide 4-[5-(1-adamantyl)-2-fluoro-4-méthoxybenzoyloxy] benzoïque
- l'acide 4-[3,5-di-tert-butyl-4-hydroxybenzoyloxy]benzoïque
- l'acide 4-[3-(1-adamantyl)-4-vinylbenzoyloxy]benzoïque
- l'acide 4-[3-(1-adamantyl)-4-éthylbenzoyloxy]benzoïque
- l'acide 4-[3-(1-adamantyl)-4-allyloxybenzoyloxy]benzoïque
- l'acide 4-[3-(1-adamantyl)-4-méthylthiobenzoyloxy]benzoïque
- l'acide 4-(5,6,7,8-tétrahydro-5,5,8,8-tétraméthyl-2-naphtylglyoxyloyloxy)benzoïque
- l'acide 4-(5,6,7,8-tétrahydro-5,5,8,8-tétraméthyl-2-naphtoyloxyméthyl)benzoïque
- l'acide 4-(3,5-di-tert-butyl-4-hydroxybenzoyloxyméthyl)benzoïque
- l'acide 4-(3-tert-butyl-4-méthoxybenzoyloxyméthyl) benzoïque
- l'acide 5-(5,6,7,8-tétrahydro-5,5,8,8-tétraméthyl-2-naphtoyloxyméthyl)-2-thiophènecarboxylique
- l'acide 4-(5,6,7,8-tétrahydro-5,5,8,8-tétraméthyl-2-naphtoylméthyloxy) benzoïque
- l'acide 4-[1-(5,6,7,8-tétrahydro-5,5,8,8-tétraméthyl-2-naphtoyloxy) éthyl]benzoïque
- l'acide 4-[[(5,6,7,8-tétrahydro-5,5,8,8-tétraméthyl-2-naphtyl) éthyloxy]carbonyl]benzoïque
- l'acide 4-(5,6,7,8-tétrahydro-5,5,8,8-tétraméthyl-2-naphtoylméthylamino) benzoïque
- l'acide 4-(5,6,7,8-tétrahydro-5,5,8,8-tétraméthyl-2-naphtoyl formamido) benzoïque
- l'acide 4-(α-hydroxy-5,6,7,8-tétrahydro-5,5,8,8-tétraméthyl-2-naphtylacétamido)benzoïque
- l'acide 4-(α-fluoro-5,6,7,8-tétrahydro-5,5,8,8-tétraméthyl-2-naphtylacétamido) benzoïque
- l'acide 6-[3-(1-adamantyl)-4-(2,3-dihydroxypropyloxy) phényl]-2-naphtoïque
- l'acide 6-[3-(1-adamantyl)-4-(3-hydroxypropyloxy) phényl]-2-naphtoïque
- l'acide 6-[3-(1-adamantyl)-4-acétoxyméthylphényl]-2-naphtoïque
- l'acide 6-[3-(1-adamantyl)-4-méthoxycarbonylphényl]-2-naphtoïque
- l'acide 6-[3-(1-adamantyl)-4-méthoxycarbonyléthylphényl]-2-naphtoïque
- l'acide 6-[3-(1-adamantyl)-4-(2-hydroxypropyl) phényl]-2-naphtoïque
- l'acide 2-hydroxy-4-[2-hydroxy-2-(5,6,7,8-tétrahydro-5,5,8,8-tétraméthyl-2-naphtyl) éthoxy]benzoïque
- le 2-hydroxy-4-[2-hydroxy-2-(5,6,7,8-tétrahydro-5,5,8,8-tétraméthyl-2-naphtyl) éthoxy]benzoate de méthyle
- l'acide 2-hydroxy-4-[2-hydroxyimino-2-(5,6,7,8-tétrahydro-5,5,8,8-tétraméthyl-2-naphtyl) éthoxy]benzoïque
- l'acide 2-acétyloxy-4-[2-acétyloxy-2-(5,6,7,8-tétrahydro-5,5,8,8-tétraméthyl-2-naphtyl) éthoxy]benzoïque
- l'acide 2-hydroxy-4-[2-acétyloxy-2-(5,6,7,8-tétrahydro-5,5,8,8-tétraméthyl-2-naphtyl) éthoxy]benzoïque
- l'acide 2-acétyloxy-4-[2-hydroxy-2-(5,6,7,8-tétrahydro-5,5,8,8-tétraméthyl-2-naphtyl) éthoxy]benzoïque
- l'acide 4-(N-méthyl-5,6,7,8-tétrahydro-5,5,8,8-tétraméthyl-2-naphtylcarboxamidino)benzoïque
- l'acide 4-(5,6,7,8-tétrahydro-5,5,8,8-tétraméthyl-2-naphtylcarboxamidino) benzoïque.

3. Composition selon la revendication 1 ou 2, caractérisée par le fait que ledit stérol inhibiteur est choisi parmi les composés ayant le squelette hydrocarboné du cholestérol ou du lanostérol et portant en outre au moins un substituant alkylé, oxygéné et/ou azoté, et/ou au moins une insaturation.

4. Composition selon l'une quelconque des revendications précédentes, caractérisée par le fait que ledit stérol inhibiteur de la synthèse du cholestérol est un inhibiteur de la 3-hydroxy-3-méthyl-glutarylcoenzyme A réductase.

5. Composition selon la revendication 4, caractérisée par le fait que ledit stérol inhibiteur est choisi parmi les stérols portant sur la structure cholestérol ou sur une structure dérivée au moins un substituant oxygéné autre que l'hydroxyle en position 3, et le 6-nitrocholestérol

6. Composition selon la revendication 5, caractérisée par le fait que le substituant oxygéné est un hydroxyle, un oxo, un époxy, un peroxyde ou un carbonyle en position 4, 7, 14, 15, 20, 22, 24, 25 ou 26.

7. Composition selon la revendication 6, caractérisée par le fait que ledit stérol est le 25-hydroxycholestérol.

8. Composition selon l'une quelconque des revendications 1 à 3, caractérisée par le fait que ledit inhibiteur de la biosynthèse du cholestérol est un inhibiteur de la 2,3-oxydosqualène cyclase.

9. Composition selon la revendication 8, caractérisée par le fait que ledit stérol est choisi parmi le 2-aza-2,3-dihydrosqualène ou ses dérivés, et le N-[(1,5,9)-triméthyldécyl]-4 α,10-diméthyl-8-aza-trans-décal-3 β-ol.

10. Composition selon l'une quelconque des revendications 1 à 3, caractérisée par le fait que ledit stérol est un inhibiteur du métabolisme du 24,25-dihydrolanostérol.

11. Composition selon la revendication 10, caractérisée par le fait que ledit stérol est choisi parmi :
   - le 7-oxolanost-8-en-3 β-ol,
   - le 3 β-acétoxylanost-8-en-7-one,
   - le 7-oxolanost-5,8,11-trien-3 β-ol.

12. Composition selon l'une quelconque des revendications 1 à 3, caractérisée par le fait que ledit stérol est un inhibiteur de la conversion du lanostérol en cholestérol, et en particulier de la conversion du lanostérol en desmostérol.

13. Composition selon l'une quelconque des revendications 1 à 3, caractérisée par le fait que ledit stérol est un inhibiteur de la conversion du desmostérol en cholestérol.

14. Composition selon la revendication 12 ou 13, caractérisée par le fait que ledit stérol est choisi parmi le 22-oximino cholestérol et le 20,25-diazacholestérol.

15. Composition selon l'une quelconque des revendications précédentes, caractérisée par le fait que le rétinoïde est présent en proportions pouvant aller de 0,0001 à 50 % en poids, par rapport au poids total de la composition.

16. Composition selon la revendication 15, caractérisée par le fait que le rétinoïde est présent en proportions pouvant aller de 0,0001 à 1 % en poids par rapport au poids total de la composition.

17. Composition selon la revendication 15, caractérisée par le fait que le rétinoïde est présent en proportions pouvant aller de 0,01 à 50 % en poids par rapport au poids total de la composition.

18. Composition selon l'une quelconque des revendications 16 et 17, caractérisés par le fait que le rapport de la concentration molaire du stérol à la concentration molaire du rétinoïde est dans la gamme de 0,1 à 10.

19. Composition selon l'une quelconque des revendications précédentes, caractérisée par le fait qu'elle contient, outre les ingrédients actifs, un véhicule pharmaceutique compatible avec une administration par voie topique (peau et muqueuses), oculaire,buccale ou systémique.

20. Composition selon l'une quelconque des revendications précédentes, caractérisée par le fait qu'elle se présente sous la forme d'une composition en deux parties comprenant, réunis dans un emballage approprié, d'une part un rétinoïde tel que défini dans l'une quelconque des revendications 1 et 2, dans un conteneur approprié, et, dans un second conteneur, un stérol inhibiteur de la biosynthèse du cholestérol tel que défini dans l'une quelconque des revendications 3 à 14.

21. Utilisation, en association, d'au moins un rétinoïde et d'au moins un stérol, dans la préparation d'une composition pharmaceutique destinée à traiter ou à corriger les troubles de la kératinisation épidermique, les troubles de la prolifération épidermique ou épithéliale, et/ou les troubles de la fonction sébacée, accompagnés ou non d'une composante inflammatoire et/ou immunoallergique, ou dans la préparation d'une composition cosmétique destinée à lutter contre le vieillissement cutané ou à améliorer l'aspect de la peau des personnes présentant des troubles de la kératinisation ou souffrant de séborrhée, ladite composition étant telle que définie dans l'une quelconque des revendications 1 à 20.

**Revendications pour l'Etat contractant suivant : ES, GR**

1. Procédé de préparation d'une composition pharmaceutique ou cosmétique, caractérisé par le fait que l'on utilise comme ingrédients actifs:
   - au moins un rétinoïde capable d'inhiber l'expression de la transglutaminase membranaire,
   - et au moins un stérol inhibiteur de la biosynthèse du cholestérol, et que l'on ajoute auxdits ingrédients actifs un véhicule compatible avec une administration par voie topique, oculaire ou systémique.

2. Procédé selon la revendication 1, caractérisé par le fait que ledit rétinoïde est choisi parmi :
   - Acide rétinoïque all-trans ou 13-cis,
   - Acide (all-E)-9-(4-méthoxy-2,3,6-triméthylphényl)-3,7-diméthyl-2,4,6,8-nonatétraénoïque,
   - Acide (E,E,E)-7-(2,3-dihydro-1,1,3,3-tétraméthyl-IH-indèn-5-yl)-3,7-diméthyl-2,4,6-octatriènoïque,
   - Acide (E,E,E)-7-(5,6,7,8-tétrahydro-5,5,8,8-tétraméthyl-2-naphthalényl-3,7-diméthyl-2,4,6-octatriènoïque,
   - Acide (E)-4-[(2,3-dihydro-1,1,3,3-tétraméthyl-1H-indèn-5-yl)-1-propènyl] benzoïque,
   - Acide (E)-4-[2-(5,6,7,8-tétrahydro-5,5,8,8-tétraméthyl-2-naphtalènyl)-1-propènyl]benzoïque,
   - Acide 2-(5,6,7,8-tétrahydro-5,5,8,8-tétraméthyl-2-naphtyl)-6-naphtalène carboxylique,
   - Acide (E)-4-[2-(5,6,7,8-tétrahydro-5,5,8,8-tétraméthyl-2-naphtalènyl)-1-propènyl] benzène sulfonique
   - Acide (E,E)-4-[2-méthyl-4-(2,6,6-triméthyl-1-cyclohexèn-1-yl)-1,3-butadiènyl]benzoïque,
   - Acide (E,E)-4-[4-méthyl-6-(2,6,6-triméthyl-1-cyclohexèn-1-yl)-1,3,5-hexatriènyl]benzoïque,
   - Acide (E)-6-[2-(2,6,6-triméthyl-1-cyclohexèn-1-yl)-éthènyl]-2-naphthalènecarboxylique,
   - Acide (E,E)-5-[2-méthyl-4-(2,6,6-triméthyl-1-cyclohexèn-1-yl)-1,3-butadiènyl]-2-thiophène carboxylique,
   - Acide (E)-4-[2-(5,6,7,8-tétrahydro-8,8-diméthyl-2-naphtalènyl)-1-propènyl]benzoïque,
   - Acide 4-[(5,6,7,8-tétrahydro-5,5,8,8-tétraméthyl-2-naphtalènyl) éthynyl]benzoïque,
   - Acide (E)-4-[2-(5,6,7,8-tétrahydro-3-méthyl-5,5,8,8-tétraméthyl-2-naphtalènyl)-1-propènyl]benzoïque,
   - Acide 4-(5,6,7,8-tétrahydro-5,5,8,8-tétraméthyl-2-naphtalènylcarbamoyl) benzoïque,
   - Acide 4-(5,6,7,8-tétrahydro-5,5,8,8-tétraméthyl-2-naphtamido) benzoïque,
   - Acide (E)-4-[3-(3,5 di-tert-butylphényl)-3-oxo-1-propènyl] benzoïque,
   - Acide 6-[(5,6,7,8-tétrahydro-5,5,8,8-tétraméthyl-2-naphtalènyl) éthynyl]3-pyridine carboxylique,
   - Acide 2-(5,6,7,8-tétrahydro-5,5,8,8-tétraméthyl-2-naphtyl)-6-benzo(b) thiophène carboxylique,
   - Acide 2-(5,6,7,8-tétrahydro-5,5,8,8-tétraméthyl-2-naphtyl)-6-benzo(b) furanecarboxylique,
   - Acide 2-(5,6,7,8-tétrahydro-5,5,8,8-tétraméthyl-2-naphtyl)-6-indole carboxylique,
   - Acide 2-[3-(1-adamantyl)-4-méthoxyphényl]-5 benzimidazole carboxylique,
   - Acide 2-(5,6,7,8-tétrahydro-5,5,8,8-tétraméthyl-2-naphtyl)-5-benzimidazole carboxylique,
   - Acide 4-(5,6,7,8-tétrahydro-5,5,8,8-tétraméthyl-2-anthracényl) benzoïque,
   - Acide 6-[3-(1-adamantyl)-4-méthoxyphényl]-2-naphtoïque,
   - Acide 4-[3-(1-adamantyl)-4-méthoxybenzamido]benzoïque,
   - Acide 4-[3-(1-adamantyl)-4-méthoxybenzamido]benzoïque,
   - Acide 4-[3-(1-adamantyl)-4-méthoxy benzoylthio]benzoïque,
   - Acide 4-[3-(1-adamantyl)-4-méthoxy benzoyloxy]benzoïque,
   - Acide 2-(5,6,7,8-tétrahydro-5,5,8,8-tétraméthyl-2-naphtyl) 6-carbonyl naphtalène carboxylique,
   - Acide p-(5,6,7,8-tétrahydro-5,5,8,8-tétraméthyl-2-1H-benz[f]indolyl) benzoïque,
   - Acide trans-(5,5,8,8-tétraméthyl-5,6,7,8 tétrahydro-2-naphtyl)-4-carbonyl-$\alpha$-méthyl cinnamique,
   - la vitamine A ou rétinol, ainsi que ses esters, tels que l'acétate, le propionate ou le palmitate de rétinol
   - l'aldéhyde de vitamine A ou rétinal
   - l'acide 4-[3-(1-adamantyl)-4-méthoxybenzoyloxy]-2-fluorobenzoïque
   - l'acide 4-[3-(1-adamantyl)-4-méthoxybenzoyloxy]-2-methylbenzoïque
   - l'acide 4-[3-(1-adamantyl)-4-méthoxybenzoyloxy]-2-hydroxybenzoïque
   - l'acide 4-[5-(1-adamantyl)-2-fluoro-4-méthoxybenzoyloxy] benzoïque
   - l'acide 4-[3,5-di-tert-butyl-4-hydroxybenzoyloxy]benzoïque
   - l'acide 4-[3-(1-adamantyl)-4-vinylbenzoyloxy]benzoïque

- l'acide 4-[3-(1-adamantyl)-4-allyloxybenzoyloxy]benzoïque
- l'acide 4-[3-(1-adamantyl)-4-méthylthiobenzoyloxy]benzoïque
- l'acide 4-[3(1-adamantyl)-4-méthylthiobenzoyloxy]benzoïque
- l'acide 4-(5,6,7,8-tétrahydro-5,5,8,8-tétraméthyl-2-naphtylglyoxyloyloxy) benzoïque
- l'acide 4-(5,6,7,8-tétrahydro-5,5,8,8-tétraméthyl-2-naphtoyloxyméthyl) benzoïque
- l'acide 4-(3,5-di-tert-butyl-4-hydroxybenzoyloxyméthyl) benzoïque
- l'acide 4-(3-tert-butyl-4-méthoxybenzoyloxyméthyl) benzoïque
- l'acide 5-(5,6,7,8-tétrahydro-5,5,8,8-tétraméthyl-2-naphtoyloxyméthyl)-2-thiophènecarboxylique
- l'acide 4-(5,6,7,8-tétrahydro-5,5,8,8-tétraméthyl-2-naphtoylméthyloxy) benzoïque
- l'acide 4-[1-(5,6,7,8-tétrahydro-5,5,8,8-tétraméthyl-2-naphtoyloxy) éthyl]benzoïque
- l'acide 4-[[(5,6,7,8-tétrahydro-5,5,8,8-tétraméthyl-2-naphtyl) éthyloxy]carbonyl]benzoïque
- l'acide 4-(5,6,7,8-tétrahydro-5,5,8,8-tétraméthyl-2-naphtoylméthylamino) benzoïque
- l'acide 4-(5,6,7,8-tétrahydro-5,5,8,8-tétraméthyl-2-naphtoyl formamido) benzoïque
- l'acide 4-(α-hydroxy-5,6,7,8-tétrahydro-5,5,8,8-tétraméthyl-2-naphtoyl-2-naphtylacétamido) benzoïque
- l'acide 4-(α-fluoro-5,6,7,8-tétrahydro-5,5,8,8-tétraméthyl-2-naphtylacétamido) benzoïque
- l'acide 6-[3-(1-adamantyl)-4-(2,3-dihydroxypropyloxy) phényl]-2-naphtoïque
- l'acide 6-[3-(1-adamantyl)-4-(3-hydroxypropyloxy) phényl]-2-naphtoïque
- l'acide 6-[3-(1-adamantyl)-4-acétoxyméthylphényl]-2-naphtoïque
- l'acide 6-[3-(1-adamantyl)-4-méthoxycarbonylphényl]-2- naphtoïque
- l'acide 6-[3-(1-adamantyl)-4-méthoxycarbonyléthylphényl] -2-naphtoïque
- l'acide 6-[3-(1-adamantyl)-4-(2-hydroxypropyl) phényl] -2-naphtoïque
- l'acide 2-hydroxy-4-[2-hydroxy-2-(5,6,7,8-tétrahydro-5,5,8,8-tétraméthyl-2-naphtyl) éthoxy]benzoïque
- le 2-hydroxy-4-[2-hydroxy-2-(5,6,7,8-tétrahydro-5,5,8,8-tétraméthyl-2-naphtyl) éthoxy]benzoate de méthyle
- -l'acide 2-hydroxy-4-[2-hydroxyimino-2-(5,6,7,8-tétrahydro-5,5,8,8-tétraméthyl-2-naphtyl) éthoxy]benzoïque
- -l'acide 2-acétyloxy-4-[2-acétyloxy-2-(5,6,7,8-tétrahydro-5,5,8,8-tétraméthyl-2-naphtyl) éthoxy]benzoïque
- l'acide 2-hydroxy-4-[2-acétyloxy-2-(5,6,7,8-tétrahydro-5,5,8,8-tétraméthyl-2-naphtyl) éthoxy]benzoïque
- l'acide 2-acétyloxy-4-[2-hydroxy-2-(5,6,7,8-tétrahydro-5,5,8,8-tétraméthyl-2-naphtyl) éthoxy]benzoïque
- l'acide 4-(N-méthyl-5,6,7,8-tétrahydro-5,5,8,8-tétraméthyl-2-naphtylcarboxamidino) benzoïque
- l'acide 4-(5,6,7,8-tétrahydro-5,5,8,8-tétraméthyl-2-naphtylcarboxamidino) benzoïque.

3. Procédé selon la revendication 1 ou 2, caractérisé par le fait que ledit stérol inhibiteur est choisi parmi les composés ayant le squelette hydrocarboné du cholestérol ou du lanostérol et portant en outre au moins un substituant alkylé, oxygéné et/ou azoté, et/ou au moins une insaturation.

4. Procédé selon l'une quelconque des revendications précédentes, caractérisé par le fait que ledit stérol inhibiteur de la synthèse du cholestérol est un inhibiteur de la 3-hydroxy-3-méthylglutarylcoenzyme A réductase.

5. Procédé selon la revendication 4, caractérisé par le fait que ledit stérol inhibiteur est choisi parmi les stérols portant sur la structure cholestérol ou sur une structure dérivée au moins un substituant oxygéné autre que l'hydroxyle en position 3, et le 6-nitrocholestérol

6. Procédé selon la revendication 5, caractérisé par le fait que le substituant oxygéné est un hydroxyle, un oxo, un époxy, un peroxyde ou un carbonyle en position 4, 7, 14, 15, 20, 22, 24, 25 ou 26.

7. Procédé selon la revendication 6, caractérisé par le fait que ledit stérol est le 25-hydroxycholestérol.

8. Procédé selon l'une quelconque des revendications 1 à 3, caractérisé par le fait que ledit inhibiteur de la biosynthèse du cholestérol est un inhibiteur de la 2,3-oxydosqualène cyclase.

9. Procédé selon la revendication 8, caractérisé par le fait que ledit stérol est choisi parmi le 2-aza-2,3-dihydrosqualène ou ses dérivés, et le N-[(1,5,9)-triméthyldécyl]-4 α,10-diméthyl-8-aza-trans- décal-3 β-ol.

10. Procédé selon l'une quelconque des revendications 1 à 3, caractérisé par le fait que ledit stérol est un inhibiteur du métabolisme du 24,25-dihydrolanostérol.

11. Procédé selon la revendication 10, caractérisé par le fait que ledit stérol est choisi parmi :

- le 7-oxolanost-8-en-3 β-ol,
- le 3 β-acétoxylanost-8-en-7-one,
- le 7-oxolanost-5,8,11-trien-3 β-ol.

**12.** Procédé selon l'une quelconque des revendications 1 à 3, caractérisé par le fait que ledit stérol est un inhibiteur de la conversion du lanostérol en cholestérol, et en particulier de la conversion du lanostérol en desmostérol.

**13.** Procédé selon l'une quelconque des revendications 1 à 3, caractérisé par le fait que ledit stérol est un inhibiteur de la conversion du desmostérol en cholestérol.

**14.** Procédé selon la revendication 12 ou 13, caractérisé par le fait que ledit stérol est choisi parmi le 22-oximino cholestérol et le 20,25-diazacholestérol.

**15.** Procédé selon l'une quelconque des revendications précédentes, caractérisé par le fait que l'on utilise le retinoïde en proportions pouvant aller de 0,0001 à 50 % en poids, par rapport au poids total de la composition.

**16.** Procédé selon la revendication 15, caractérisé par le fait que l'on utilise le rétinoïde en proportions pouvant aller de 0,0001 à 1 % en poids par poids total de la composition.

**17.** Procédé selon la revendication 15, caractérisé par le fait que l'on utilise le rétinoïde en proportions pouvant aller de 0,01 à 50 % en poids par rapport au poids total de la composition.

**18.** Procédé selon l'une quelconque des revendications 16 et 17, caractérisés par le fait que le rapport de la concentration molaire du stérol à la concentration molaire du rétinoïde est dans la gamme de 0,1 à 10.

**19.** Procédé selon l'une quelconque des revendications, précédentes, caractérisé par le fait que l'on prépare la composition sous la forme d'une composition en deux parties comprenant, réunis dans un emballage approprié, d'une part un rétinoïde tel que défini dans l'une quelconque des revendications 1 et 2, dans un conteneur approprié, et, dans un second conteneur, un stérol inhibiteur de la biosynthèse du cholestérol tel que défini dans l'une quelconque des revendications 3 à 14.

**20.** Utilisation, en association, d'au moins un rétinoïde et d'au moins un stérol, dans la préparation d'une composition pharmaceutique destinée à traiter ou à corriger les troubles de la kératinisation épidermique, les troubles de la prolifération épidermique ou épithéliale, et/ou les troubles de la fonction sébacée, accompagnés ou non d'une composante inflammatoire et/ou immunoallergique, ou dans la préparation d'une composition cosmétique destinée à lutter contre le vieillissement cutané ou à améliorer l'aspect de la peau des personnes présentant des troubles de la kératinisation ou souffrant de séborrhée, ladite composition étant telle que définie dans l'une quelconque des revendications 1 à 19.

## Patentansprüche

### Patentansprüche für folgende Vertragsstaaten : AT, BE, CH, DE, DK, FR, GB, IT, LI, LU, NL, SE

**1.** Pharmazeutische oder kosmetische Zubereitung, dadurch gekennzeichnet, daß sie eine Mischung aus
- mindestens einem Retinoid, welches in der Lage ist, die Expression der membranständigen Transglutaminase zu inhibieren, und
- mindestens einem Steroidinhibitor der Cholesterinbiosynthese enthält.

**2.** Zubereitung gemäß Anspruch 1, dadurch gekennzeichnet, daß das Retinoid ausgewählt ist aus:
- (all-trans oder 13-cis) Retinsäure,
- (all-E)-9-(4-Methoxy-2,3,6-trimethylphenyl)-3,7-dimethyl-2,4,6,8-nonatetraensäure,
- (E,E,E)-7-(2,3-Dihydro-1,1,3,3-tetramethyl-1H-inden-5-yl)-3,7-dimethyl-2,4,6-octatriensäure,
- (E,E,E)-7-(5,6,7,8-Tetrahydro-5,5,8,8-tetramethyl-2-naphtalenyl)-3,7-dimethyl-2,4,6-octatriensäure,
- (E)-4-[-(2,3-Dihydro-1,1,3,3-tetramethyl-1H-inden-5-yl)-1-propenyl]-benzoesäure,
- (E)-4-[2-(5,6,7,8-Tetrahydro-5,5,8,8,-tetramethyl-2-naphtalenyl)-1-propenyl]-benzoesäure,
- 2-(5,6,7,8-Tetrahydro-5,5,8,8-tetramethyl-2-naphtyl)-6-naphtalencarbonsäure,

- (E)-4-[2-(5,6,7,8-Tetrahydro-5,5,8,8-tetramethyl-2-naphtalenyl)-1-propenyl]-benzosulfonsäure,
- (E,E)-4-[2-Methyl-4-(2,6,6-trimethyl-1-cyclohexen-1-yl)-1,3-butadienyl]-benzoesäure,
- (E,E)-4-[4-Methyl-6-(2,6,6-trimethyl-1-cyclohexen-1-yl)-1,3,5-hexatrienyl]-benzoesäure,
- (E)-6-[2-(2,6,6-Trimethyl-1-cyclohexen-1-yl)-ethenyl]-2-naphtalencarbonsäure,
- (E,E)-5-[2-Methyl-4-(2,6,6-trimethyl-1-cyclohexen-1-yl)-1,3-butadienyl]-2-thiophencarbonsäure
- (E)-4-[2-(5,6,7,8-Tetrahydro-8,8-dimethyl-2-naphtalenyl)-1-propenyl]-benzoesäure,
- 4-[(5,6,7,8-Tetrahydro-5,5,8,8-tetramethyl-2-naphtalenyl)-ethenyl]-benzoesäure,
- (E)-4-[2-(5,6,7,8-Tetrahydro-3-methyl-5,5,8,8-tetramethyl-2-naphtalenyl)-1-propenyl]-benzoesäure,
- 4-(5,6,7,8-Tetrahydro-5,5,8,8-tetramethyl-2-naphtalenylcarbamoyl)-benzoesäure
- 4-(5,6,7,8-Tetrahydro-5,5,8,8-tetramethyl-2-naphtamido)-benzoesäure,
- (E)-4-[3-(3,5-Di-tert.-butylphenyl)-3-oxo-1-propenyl]-benzoesäure,
- 6-[(5,6,7,8-Tetrahydro-5,5,8,8-tetramethyl-2-naphtalenyl)-ethenyl]-3-pyridincarbonsäure,
- 2-(5,6,7,8-Tetrahydro-5,5,8,8-tetramethyl-2-naphtyl)-6-benzo(b)-thiophencarbonsäure,
- 2-(5,6,7,8-Tetrahydro-5,5,8,8-tetramethyl-2-naphtyl)-6-benzo(b)-furancarbonsäure,
- 2-(5,6,7,8-Tetrahydro-5,5,8,8-tetramethyl-2-naphtyl)-6-indolcarbonsäure,
- 2-[3-(1-Adamantyl)-4-methoxyphenyl]-5-benzimidazolcarbonsäure,
- 2-(5,6,7,8-Tetrahydro-5,5,8,8-tetramethyl-2-naphtyl)-5-benzimidazolcarbonsäure,
- 4-(5,6,7,8-Tetrahydro-5,5,8,8-tetramethyl-2-anthracenyl)-benzoesäure,
- 6-[3-(1-Adamantyl)-4-methoxyphenyl]-2-naphtoesäure,
- 4-[3-(1-Adamantyl)-4-methoxybenzamido]-benzoesäure,
- 4-[3-(1-Adamantyl)-4-methoxybenzoylthio]-benzoesäure,
- 4-[3-(1-Adamantyl)-4-methoxybenzoyloxy]-benzoesäure,
- 2-(5,6,7,8-Tetrahydro-5,5,8,8-tetramethyl-2-naphtyl)-6-carbonylnaphtalencarbonsäure,
- p-(5,6,7,8-Tetrahydro-5,5,8,8-tetramethyl-2-1H-benz[f]-indolyl)-benzoesäure,
- trans-(5,6,7,8-Tetrahydro-5,5,8,8-tetramethyl-2-naphtyl)-4-carbonyl-α-methylzimtsäure,
- Vitamin A oder Retinol sowie dessen Ester, beispielsweise das Acetat, Propionat oder Palmitat,
- Vitamin A-Aldehyd oder Retinal,
- 4-[3-(1-Adamantyl)-4-methoxybenzoyloxy]-2-fluorbenzoesäure,
- 4-[3-(1-Adamantyl)-4-methoxy]-2-methylbenzoesäure,
- 4-[3-(1-Adamantyl)-4-methoxybenzoyloxy]-2-hydroxybenzoesäure,
- 4-[3-(1-Adamantyl)-2-flour-4-methoxybenzoyloxy]-benzoesäure,
- 4-[3,5-Di-tert.-butyl-4-hydroxybenzoyloxy]-benzoesäure,
- 4-[3-(1-Adamantyl)-4-vinylbenzoyloxy]-benzoesäure,
- 4-[3-(1-Adamantyl)-4-ethylbenzoyloxy]-benzoesäure,
- 4-[3-(1-Adamantyl)-4-allylbenzoyloxy]-benzoesäure,
- 4-[3-(1-Adamantyl)-4-methylthiobenzoyloxy]-benzoesäure,
- 4-(5,6,7,8-Tetrahydro-5,5,8,8-tetramethyl-2-naphtylglyoxyloyloxy)-benzoesäure,
- 4-(5,6,7,8-Tetrahydro-5,5,8,8-tetramethyl-2-naphtoylmethoxy)-benzoesäure,
- 4-[3,5-Di-tert.-butyl-4-hydroxybenzoyloxymethyl]-benzoesäure,
- 4-[3-tert.-Butyl-4-methoxybenzoyloxymethyl]-benzoesäure,
- 4-(5,6,7,8-Tetrahydro-5,5,8,8-tetramethyl-2-naphtoylmethoxy)-2-thiophencarbonsäure,
- 4-(5,6,7,8-Tetrahydro-5,5,8,8-tetramethyl-2-naphtoylmethoxy)-benzoesäure,
- 4-[1-(5,6,7,8-Tetrahydro-5,5,8,8-tetramethyl-2-naphtoyloxy)-ethyl]-benzoesäure,
- 4-{[(5,6,7,8-Tetrahydro-5,5,8,8-tetramethyl-2-naphtoyloxy)-ethyloxy]-carbonyl}-benzoesäure
- 4-(5 6,7,8-Tetrahydro-5,5,8,8-tetramethyl-2-naphtoylmethylamido)-benzoesäure,
- 4-(5,6,7,8-Tetrahydro-5,5,8,8-tetramethyl-2-naphtoylformamido)-benzoesäure,
- 4-(α-Hydroxy-5,6,7,8-tetrahydro-5,5,8,8-tetramethyl-2-naphtylacetamido)-benzoesäure
- 4-(α-Fluor-5,6,7,8-tetrahydro-5,5,8,8-tetramethyl-2-naphtylacetamido)-benzoesäure,
- 6-[3-(1-Adamantyl)-4-(2,3-dihydroxypropyloxy)-phenyl]-2-naphtoesäure,
- 6-[3-(1-Adamantyl)-4-(3-hydroxypropyloxy)-phenyl]-2-naphtoesäure
- 6-[3-(1-Adamantyl)-4-acetoxymethylphenyl]-2-naphtoesäure,
- 6-[3-(1-Adamantyl)-4-methoxycarbonylphenyl]-2-naphtoesäure,
- 6-[3-(1-Adamantyl)-4-methoxycarbonylethylphenyl]-2-naphtoesäure,
- 6-[3-(1-Adamantyl)-4-(2-hydroxypropyl)-phenyl]-2-naphtoesäure,
- 2-Hydroxy-4-[2-hydroxy-2-(5,6,7,8-tetrahydro-5,5,8,8-tetramethyl-2-naphtyl)-ethoxy]-benzoesäure,
- 2-Hydroxy-4-[2-hydroxy-2-(5,6,7,8-tetrahydro-5,5,8,8-tetramethyl-2-naphtyl)-ethoxy]-benzoesäure methylester,
- 2-Hydroxy-4-[2-hydroxyimino-2-(5,6,7,8-tetrahydro-5,5,8,8-tetramethyl-2-naphtyl)-ethoxy]-benzoesäure,

- 2-Acethyloxy-4-[2-acethyloxy-2-(5,6,7,8-tetrahydro-5,5,8,8-tetramethyl-2-naphtyl)-ethoxy]-benzoesäure,
- 2-Hydroxy-4-[2-acethyloxy-2-(5,6,7,8-tetrahydro-5,5,8,8-tetramethyl-2-naphtyl)-ethoxy]-benzoesäure,
- 2-Acethyloxy-4-[2-hydroxy-2-(5,6,7,8-tetrahydro-5,5,8,8-tetramethyl-2-naphtyl)-ethoxy]-benzoesäure,
- 4-(N-Methyl-5,6,7,8-tetrahydro-5,5,8,8-tetramethyl-2-naphtylcarboxamidino)-benzoesäure und
- 4-(5,6,7,8-Tetrahydro-5,5,8,8-tetramethyl-2-naphtylcarboxamidino)-benzoesäure

**3.** Zubereitung gemäß Anspruch 1 oder 2, dadurch gekennzeichnet, daß der Steroidinhibitor aus Verbindungen mit dem Kohlenstoffskelett des Cholesterins oder des Lanosterins ausgewählt ist und zusätzlich mindestens einen weiteren Alkyl-, Sauerstoff und/oder Stickstoffsubstituenten und/oder mindestens eine ungesättigte Bindung aufweist.

**4.** Zubereitung gemäß einem der vorstehenden Ansprüche, dadurch gekennzeichnet, daß der Steroidinhibitor der Cholesterinbiosynthese ein Inhibitor der 3-Hydroxy-3-methylglutaryl-CoA-Reduktase ist.

**5.** Zubereitung gemäß Anspruch 4, dadurch gekennzeichnet, daß der Steroidinhibitor ausgewählt ist aus Steroiden, die an der Cholesterinstruktur oder einer abgeleiteten Struktur mindestens einen Sauerstoffsubstituenten oder auch eine Hydroxylgruppe in 3 Stellung tragen, und dem 6-Nitrocholesterin.

**6.** Zubereitung gemäß Anspruch 5, dadurch gekennzeichnet, daß der Sauerstoffsubstituent eine Hydroxyl-, Oxo-, Epoxy-, Peroxid- oder Carbonylgruppe in der 4, 7, 14, 15, 20, 22, 24, 25 oder 26-Stellung ist.

**7.** Zubereitung gemäß Anspruch 6, dadurch gekennzeichnet, daß das Steroid 25-Hydroxycholesterin ist.

**8.** Zubereitung gemäß einem der Ansprüche 1 bis 3, dadurch gekennzeichnet, daß der Inhibitor der Cholesterinbiosynthese ein Inhibitor der 2,3-Oxosqualencyclase ist.

**9.** Zubereitung gemäß Anspruch 8, dadurch gekennzeichnet, daß das Steroid aus 2-Aza-2,3-dihydrosqualen oder seinen Derivaten sowie dem N-[1,5,9-Trimethyldecyl]-4$\alpha$,10-dimethyl-8-aza-transdecal-3$\beta$-ol ausgewählt ist.

**10.** Zubereitung gemäß einem der Ansprüche 1 bis 3, dadurch gekennzeichnet, daß das Steroid ein Inhibitor des Metabolismus von 24,25-Dihydrolanosterin ist.

**11.** Zubereitung gemäß Anspruch 10, dadurch gekennzeichnet, daß das Steroid usgewählt ist aus 7-Oxolanost-8-en-3$\beta$-ol, 3$\beta$-Acetoxylanost-8-en-7-on und 7-Oxolanost-5,8,11-trien-3$\beta$-ol.

**12.** Zubereitung gemäß einem der Ansprüche 1 bis 3, dadurch gekennzeichnet, daß das Steroid ein Inhibitor der Umwandlung von Lanosterin in Cholesterin, insbesondere der Umwandlung von Lanosterin in Desmosterin ist.

**13.** Zubereitung gemäß einem der Ansprüche 1 bis 3, dadurch gekennzeichnet, daß das Steroid ein Inhibitor der Umwandlung von Desmosterin in Cholesterin ist.

**14.** Zubereitung gemäß einem der Ansprüche 12 oder 13, dadurch gekennzeichnet, daß das Steroid ausgewählt ist aus 22-Oximinocholesterin und 20,25-Diazacholesterin.

**15.** Zubereitung gemäß einem der vorstehenden Ansprüche, dadurch gekennzeichnet, daß das Retinoid in Konzentrationen vorliegt, welche von 0.0001 bis 50 Gew.-%, bezogen auf das Gesamtgewicht der Zubereitung, variieren können.

**16.** Zubereitung gemäß Anspruch 15, dadurch gekennzeichnet, daß das Retinoid in Konzentrationen vorliegt, welche von 0.0001 bis 1 Gew.-%, bezogen auf das Gesamtgewicht der Zubereitung, variieren können.

**17.** Zubereitung gemäß Anspruch 15, dadurch gekennzeichnet, daß das Retinoid in Konzentrationen vorliegt, welche von 0.01 bis 50 Gew.-%, bezogen auf das Gesamtgewicht der Zubereitung, variier können.

**18.** Zubereitung gemäß einem der Ansprüche 16 und 17, dadurch gekennzeichnet, daß die molare Konzentration des Steroids bezogen auf die molare Konzentration des Retinoids zwischen 0,1 und 10 liegt.

**19.** Zubereitung gemäß einem der vorstehenden Ansprüche, dadurch gekennzeichnet, daß sie neben den aktiven Substanzen einen geeigneten pharmazeutischen Träger enthält, der mit der topischen (Haut oder

Schleimhäute), bukkalen, ophtalmologischen oder systemischen Applikation vereinbar ist.

20. Zubereitung gemäß einem der vorstehenden Ansprüche, dadurch gekennzeichnet, daß sie in Form einer Zubereitung aus zwei Anteilen vorliegt, die in einer geeigneten Verpackung vereint sind, wobei der eine Teil ein gemäß einem der Ansprüche 1 und 2 definiertes Retinoid in einem geeigneten Behälter und der andere Teil ein gemäß irgendeinem der Ansprüche 3 bis 14 definierter Steroidinhibitor der Cholesterinbiosynthese in einem zweiten Behälter darstellt.

21. Verwendung von mindestens einem Retinoid und mindestens einem Steroid gleichzeitig bei der Herstellung eines Arzneimittels, welches zur Behandlung oder der Besserung von Keratinisierungsstörungen der Epidermis, anderen Störungen sowie anderen Defekten oder Überfunktionen der Epidermis- oder Epithelproliferation und/oder Funktionsstörungen der Talgdrüsen, gegebenenfalls von entzündlichen und/oder immunoallergischen Komponenten begleitet, bestimmt ist, wobei das Arzneimittel gemäß irgendeinem der Ansprüche 1 bis 20 definiert ist.

**Patentansprüche für folgenden Vertragsstaat : ES, GR**

1. Verfahren zur Herstellung einer pharmazeutischen oder kosmetischen Zubereitung, dadurch gekennzeichnet, daß sie als aktive Inhaltsstoffe enthält:
   - mindestens einem Retinoid, welches in der Lage ist, die Expression der membranständigen Transglutaminase zu inhibieren,
   - mindestens einem Steroidinhibitor der Cholesterinbiosynthese und daß die genannten aktiven Inhaltsstoffe mit einem Träger verbunden sind, der für die topische, ophtamologische oder systemische Applikation geeignet ist.

2. Verfahren gemäß Anspruch 1, dadurch gekennzeichnet, daß das Retinoid ausgewählt ist aus:
   - (all-trans oder 13-cis) Retinsäure,
   - (all-E)-9-(4-Methoxy-2,3,6-trimethylphenyl)-3,7-dimethyl-2,4,6,8-nonatetraensäure,
   - (E,E,E)-7-(2,3-Dihydro-1,1,3,3-tetramethyl-1H-inden-5-yl)-3,7-dimethyl-2,4,6-octatriensäure,
   - (E,E,E)-7-(5,6,7,8-Tetrahydro-5,5,8,8-tetramethyl-2-naphtalenyl)-3,7-dimethyl-2,4,6-octatriensäure,
   - (E)-4-[(2,3-Dihydro-1,1,3,3-tetramethyl-1H-inden-5-yl)-1-propenyl]-benzoesäure,
   - (E)-4-[2-(5,6,7,8-Tetrahydro-5,5,8,8-tetramethyl-2-naphtalenyl)-1-propenyl]-benzoesäure,
   - 2-(5,6,7,8-Tetrahydro-5,5,8,8-tetramethyl-2-naphtyl)-6-naphtalencarbonsäure,
   - (E)-4-[2-(5,6,7,8-Tetrahydro-5,5,8,8-tetramethyl-2-naphtalenyl)-1-propenyl]-benzolsulfonsäure,
   - (E,E)-4-[2-Methyl-4-(2,6,6-trimethyl-1-cyclohexen-1-yl)-1,3-butadienyl]-benzoesäure
   - (E,E)-4-[4-Methyl-6-(2,6,6-trimethyl-1-cyclohexen-1-yl)-1,3,5-hexatrienyl]-benzoesäure,
   - (E)-6-[2-(2,6,6-Trimethyl-1-cyclohexen-1-yl)-ethenyl]-2-naphtalencarbonsäure,
   - (E,E)-5-[2-Methyl-4-(2,6,6-trimethyl-1-cyclohexen-1-yl)-1,3-butadienyl]-2-thiophencarbonsäure
   - (E)-4-[2-(5,6,7,8-Tetrahydro-8,8-dimethyl-2-naphtalenyl)-1-propenyl]-benzoesäure
   - 4-[(5,6,7,8-Tetrahydro-5,5,8,8-tetramethyl-2-naphtalenyl)-ethenyl]-benzoesäure,
   - (E)-4-[2-(5,6,7,8-Tetrahydro-3-methyl-5,5,8,8-tetramethyl-2-naphtalenyl)-1-propenyl]-benzoesäure,
   - 4-(5,6,7,8-Tetrahydro-5,5,8,8-tetramethyl-2-naphtalenylcarbamoyl]-benzoesäure,
   - 4-(5 6,7,8-Tetrahydro-5 5,8,8-tetramethyl-2-naphtamido)-benzoesäure,
   - (E)-4-[3-(3,5-Di-tert.-butylphenyl)-3-oxo-1-propenyl]-benzoesäure
   - 6-[(5,6,7,8-Tetrahydro-5,5,8,8-tetramethyl-2-naphtalenyl)-ethenyl]-3-pyridincarbonsäure,
   - 2-(5,6,7,8-Tetrahydro-5,5,8,8-tetramethyl-2-naphtyl)-6-benzo(b)-thiophencarbonsäure,
   - 2-(5,6,7,8-Tetrahydro-5,5,8,8-tetramethyl-2-naphtyl)-6-benzo(b)-furancarbonsäure,
   - 2-(5,6,7,8-Tetrahydro-5,5,8,8-tetramethyl-2-naphtyl)-6-indolcarbonsäure
   - 2-[3-(1-Adamantyl)-4-methoxyphenyl]-5-benzimidazolcarbonsäure,
   - 2-(5,6,7,8-Tetrahydro-5,5,8,8,-tetramethyl-2-naphtyl)-5-benzimidazolcarbonsäure,
   - 4-(5,6,7,8-Tetrahydro-5,5,8,8-tetramethyl-2-anthracenyl)-benzoesäure,
   - 6-[3-(1-Adamantyl)-4-methoxyphenyl]-2-naphtoesäure,
   - 4-[3-(1-Adamantyl)-4-methoxybenzamido]-benzoesäure,
   - 4-[3-(1-Adamantyl)-4-methoxybenzoylthio]-benzoesäure
   - 4-[3-(1-Adamantyl)-4-methoxybenzoyloxy]-benzoesäure,
   - 2-(5,6,7,8-Tetrahydro-5,8,8,8-tetramethyl-2-naphtyl)-6-carbonylnaphtalencarbonsäure,
   - p-(5,6,7,8-Tetrahydro-5,5,8,8-tetramethyl-2-1H-benz[f]-indolyl)-benzoesäure,
   - trans-(5,6,7,8-Tetrahydro-5,5,8,8-tetramethyl-2-naphtyl)-4-carbonyl-α-methylzimtsäure,

- Vitamin A oder Retinol sowie dessen Ester, beispielsweise das Acetat, Propionat oder Palmitat,
- Vitamin A-Aldehyd oder Retinal,
- 4-[3-(1-Adamantyl)-4-methoxybenzoyloxy]-2-fluorbenzoesäure,
- 4-[3-(1-Adamantyl)-4-methoxybenzoyloxy]-2-methylbenzoesäure,
- 4-[3-(1-Adamantyl)-4-methoxybenzoyloxy]-2-hydroxybenzoesäure,
- 4-[3-(1-Adamantyl)-2-flour-4-methoxybenzoyloxy]-benzoesäure,
- 4-[3,5-Di-tert.-butyl-4-hydroxybenzoyloxy]-benzoesäure,
- 4-[3-(1-Adamantyl)-4-vinylbenzoyloxy]-benzoesäure,
- 4-[3-(1-Adamantyl)-4-ethylbenzoyloxy]-benzoesäure,
- 4-[3-(1-Adamantyl)-4-allylbenzoyloxy]-benzoesäure,
- 4-[3-(1-Adamantyl)-4-methylthiobenzoyloxy]-benzoesäure,
- 4-(5,6,7,8-Tetrahydro-5,5,8,8-tetramethyl-2-naphtylglyoxyloyloxy)-benzoesäure,
- 4-(5,6,7,8-Tetrahydro-5,5,8,8-tetramethyl-2-naphtoylmethoxy)-benzoesäure,
- 4-[3,5-Di-tert.-butyl-4-hydroxybenzoyloxymethyl]-benzoesäure,
- 4-[3-tert.-Butyl-4-methoxybenzoyloxymethyl]-benzoesäure,
- 4-(5,6,7,8-Tetrahydro-5,5,8,8-tetramethyl-2-naphtoylmethoxy)-2-thiophencarbonsäure,
- 4-(5,6,7,8-Tetrahydro-5,5,8,8-tetramethyl-2-naphtoylmethyloxy)-benzoesäure,
- 4-[1-(5,6,7,8-Tetrahydro-5,5,8,8-tetramethyl-2-naphtoyloxy)-ethyl]-benzoesäure,
- 4-{[(5,6,7,8-Tetrahydro-5,5,8,8-tetramethyl-2-naphtoyloxy)-ethyloxy]-carbonyl]-benzoesäure,
- 4-(5,6,7,8-Tetrahydro-5,5,8,8-tetramethyl-2-naphtoylmethylamido)-benzoesäure,
- 4-(5,6,7,8-Tetrahydro-5,5,8,8-tetramethyl-2-naphtoylformamido)-benzoesäure,
- 4-($\alpha$-Hydroxy-5,6,7,8-tetrahydro-5,5,8,8-tetramethyl-2-naphtylacetamido)-benzoesäure,
- 4-($\alpha$-Fluor-5,6,7,8-tetrahydro-5,5,8,8-tetramethyl-2-naphtylacetamido)-benzoesäure,
- 6-[3-(1-Adamantyl)-4-(2,3-dihydroxypropyloxy)-phenyl]-2-naphtoesäure,
- 6-[3-(1-Adamantyl)-4-(3-hydroxypropyloxy)-phenyl]-2-naphtoesäure,
- 6-[3-(1-Adamantyl)-4-acetoxymethylphenyl]-2-naphtoesäure,
- 6-[3-(1-Adamantyl)-4-methoxycarbonylphenyl]-2-naphtoesäure,
- 6-[3-(1-Adamantyl)-4-methoxycarbonylethylphenyl]-2-naphtoesäure,
- 6-[3-(1-Adamantyl)-4-(2-hydroxypropyl)-phenyl]-2-naphtoesäure,
- 2-Hydroxy-4-[2-hydroxy-2-(5,6,7,8-tetrahydro-5,5,8,8-tetramethyl-2-naphtyl)-ethoxy]-benzoesäure,
- 2-Hydroxy-4-[2-hydroxy-2-(5,6,7,8-tetrahydro-5,5,8,8-tetramethyl-2-naphtyl)-ethoxy]-benzoesäure methylester,
- 2-Hydroxy-4-[2-hydroxyimino-2-(5,6,7,8-tetrahydro-5,5,8,8-tetramethyl-2-naphtyl)-ethoxy]-benzoesäure,
- 2-Acethyloxy-4-[2-acethyloxy-2-(5,6,7,8-tetrahydro-5,5,8,8-tetramethyl-2-naphtyl)-ethoxyl-benzoesäure,
- 2-Hydroxy-4-[2-acethyloxy-2-(5,6,7,8-tetrahydro-5,5,8,8-tetramethyl-2-naphtyl)-ethoxy]-benzoesäure,
- 2-Acethyloxy-4-[2-hydroxy-2-(5,6,7,8-tetrahydro-5,5,8,8-tetramethyl-2-naphtyl)-ethoxy]-benzoesäure,
- 4-(N-Methyl-5,6,7,8-tetrahydro-5,5,8,8-tetramethyl-2-naphtylcarboxamidino)-benzoesäure und
- 4-(5,6,7,8-Tetrahydro-5,5,8,8-tetramethyl-2-naphtylcarboxamidino)-benzoesäure

**3.** Verfahren gemäß Anspruch 1 oder 2, dadurch gekennzeichnet, daß der Steroidinhibitor aus Verbindungen mit dem Kohlenstoffskelett des Cholesterins oder des Lanosterins ausgewählt ist und zusätzlich mindestens einen weiteren Alkyl-, Sauerstoff und/oder Stickstoffsubstituenten und/oder mindestens eine ungesättigte Bindung aufweist.

**4.** Verfahren gemäß einem der vorstehenden Ansprüche, dadurch gekennzeichnet, daß der Steroidinhibitor der Cholesterinbiosynthese ein Inhibitor der 3-Hydroxy-3-methylglutaryl-CoA-Reduktase ist.

**5.** Verfahren gemäß Anspruch 4, dadurch gekennzeichnet, daß der Steroidinhibitor ausgewählt ist aus Steroiden, die an der Cholesterinstruktur oder einer abgeleiteten Struktur mindestens einen Sauerstoffsubstituenten oder auch eine Hydroxylgruppe in 3 Stellung tragen, und dem 6-Nitrocholesterin.

**6.** Verfahren gemäß Anspruch 5, dadurch gekennzeichnet, daß der Sauerstoffsubstituent eine Hydroxyl-, Oxo-, Epoxy-, Peroxid- oder Carbonylgruppe in der 4, 7, 14, 15, 20, 22, 24, 25 oder 26-Stellung ist.

**7.** Verfahren gemäß Anspruch 6, dadurch gekennzeichnet, daß das Steroid 25-Hydroxycholesterin ist.

**8.** Verfahren gemäß einem der Ansprüche 1 bis 3, dadurch gekennzeichnet, daß der Inhibitor der Cholesterinbiosynthese ein Inhibitor der 2,3-Oxosqualencyclase ist.

9. Verfahren gemäß Anspruch 8, dadurch gekennzeichnet, daß das Steroid aus 2-Aza-2,3-dihydrosqualen oder seinen Derivaten sowie dem N-[1,5,9-Trimethyldecyl]-4α,10-dimethyl-8-aza-transdecal-3β-ol ausgewählt ist.

10. Verfahren gemäß einem der Ansprüche 1 bis 3, dadurch gekennzeichnet, daß das Steroid ein Inhibitor des Metabolismus von 24,25-Dihydrolanosterin ist.

11. Verfahren gemäß Anspruch 10, dadurch gekennzeichnet, daß das Steroid ausgewählt ist aus 7-Oxolanost-8-en-3β-ol, 3β-Acetoxylanost-8-en-7-on und 7-Oxolanost-5,8,11-trien-3β-ol.

12. Verfahren gemäß einem der Ansprüche 1 bis 3, dadurch gekennzeichnet, daß das Steroid ein Inhibitor der Umwandlung von Lanosterin in Cholesterin, insbesondere der Umwandlung von Lanosterin in Desmosterin ist.

13. Verfahren gemäß einem der Ansprüche 1 bis 3, dadurch gekennzeichnet, daß das Steroid ein Inhibitor der Umwandlung von Desmosterin in Cholesterin ist.

14. Verfahren gemäß einem der Ansprüche 12 oder 13, dadurch gekennzeichnet, daß das Steroid ausgewählt ist aus 22-Oximinocholesterin und 20,25-Diazacholesterin.

15. Verfahren gemäß einem der vorstehenden Ansprüche, dadurch gekennzeichnet, daß das verwendete Retinoid in Konzentrationen, welche von 0.0001 bis 50 Gew.- % gehen, bezogen auf das Gesamtgewicht der Verfahren, variieren kann.

16. Verfahren gemäß Anspruch 15, dadurch gekennzeichnet, daß das verwendete Retinoid in Konzentrationen, welche von 0.0001 bis 1 Gew.-% gehen, bezogen auf das Gesamtgewicht der Zubereitung, variieren kann.

17. Verfahren gemäß Anspruch 15, dadurch gekennzeichnet, daß das verwendete Retinoid in Konzentrationen, welche von 0.01 bis 50 Gew.-% gehen, bezogen auf das Gesamtgewicht der Zubereitung, variieren kann.

18. Verfahren gemäß einem der Ansprüche 16 und 17, dadurch gekennzeichnet, daß die molare Konzentration des Steroids bezogen auf die molare Konzentration des Retinoids zwischen 0,1 und 10 liegt.

19. Verfahren gemäß einem der vorstehenden Ansprüche, dadurch gekennzeichnet, daß man eine Zubereitung herstellt, die in Form einer Zubereitung aus zwei Anteilen vorliegt, die in einer geeigneten Verpackung vereint sind, wobei der eine Teil ein gemäß einem der Ansprüche 1 und 2 definiertes Retinoid in einem geeigneten Behälter und der andere Teil ein gemäß irgendeinem der Ansprüche 3 bis 14 definierter Steroidinhibitor der Cholesterinbiosynthese in einem zweiten Behälter darstellt.

20. Verwendung von mindestens einem Retinoid und mindestens einem Steroid gleichzeitig bei der Herstellung einer pharmazeutischen Zubereitung, welche zur Behandlung oder der Besserung von Keratinisierungsstörungen der Epidermis, Störungen der Epidermis- oder Epithelproliferation und/oder Funktionsstörungen der Talgdrüsen, gegebenenfalls von entzündlichen und/oder immunoallergischen Komponenten begleitet, bestimmt ist, oder bei der Herstellung einer kosmetischen Zubereitung, die zu einer Verwendung zur Vorbeugung von Hautalterung oder der Besserung des Aussehens der Haut bei Personen, die Störungen der Keratinisierung aufweisen oder an Seborrhoe leiden, wobei die Zubereitung gemäß irgendeinem der Ansprüche 1 bis 19 definiert ist.

**Claims**

**Claims for the following Contracting States : AT, BE, CH, DE, DK, FR, GB, IT, LI, LU, NL, SE**

1. Pharmaceutical or cosmetic composition characterized by the fact that it comprises as a combination:
   - at least one retinoid capable of inhibiting the expression of membrane transglutaminase,
   - and at least one sterol which inhibits the biosynthesis of cholesterol.

2. Composition according to Claim 1, characterized by the fact that the said retinoid is chosen from:

- all-trans- or 13-cis-retinoic acid,
- (all-E)-9-(4-methoxy-2,3,6-trimethylphenyl)-3,7-dimethyl-2,4,6,8-nonatetraenoic acid,
- (E,E,E)-7-(2,3-dihydro-1,1,3,3-tetramethyl-1H-inden-5-yl)-3,7-dimethyl-2,4,6-octatrienoic acid,
- (E,E,E)-7-(5,6,7,8-tetrahydro-5,5,8,8-tetramethyl-2-naphthalenyl-3,7-dimethyl-2,4,6-octatrienoic acid,
- (E)-4-[(2,3-dihydro-1,1,3,3-tetramethyl-1H-inden-5-yl)-1-propenyl]benzoic acid,
- (E)-4-[2-(5,6,7,8-tetrahydro-5,5,8,8-tetramethyl-2-naphthalenyl)-1-propenyl]benzoic acid,
- 2-(5,6,7,8-tetrahydro-5,5,8,8-tetramethyl-2-naphthyl)-6-naphthalenecarboxylic acid,
- (E)-4-[2-(5,6,7,8-tetrahydro-5,5,8,8-tetramethyl-2-naphthalenyl)-1-propenyl] benzenesulphonic acid,
- (E,E)-4-[2-methyl-4-(2,6,6-trimethyl-1-cyclohexen-1-yl)-1,3-butadienyl]benzoic acid,
- (E,E)-4-[4-methyl-6-(2,6,6-trimethyl-1-cyclohexen-1-yl)-1,3,5-hexatrienyl]benzoic acid,
- (E)-6-[2-(2,6,6-trimethyl-1-cyclohexen-1-yl)-ethenyl]-2-naphthalenecarboxylic acid,
- (E,E)-5-[2-methyl-4-(2,6,6-trimethyl-1-cyclohexen-1-yl)-1,3-butadienyl]-2-thiophenecarboxylic acid,
- (E)-4-[2-(5,6,7,8-tetrahydro-8,8-dimethyl-2-naphthalenyl)-1-propenyl]benzoic acid,
- 4-[(5,6,7,8-tetrahydro-5,5,8,8-tetramethyl-2-naphthalenyl)ethynyl]benzoic acid,
- (E)-4-[2-(5,6,7,8-tetrahydro-3-methyl-5,5,8,8-tetramethyl-2-naphthalenyl)-1-propenyl]benzoic acid,
- 4-(5,6,7,8-tetrahydro-5,5,8,8-tetramethyl-2-naphthalenylcarbamoyl)benzoic acid,
- 4-(5,6,7,8-tetrahydro-5,5,8,8-tetramethyl-2-naphthamido)benzoic acid,
- (E)-4-[3-(3,5-di-tert-butylphenyl)-3-oxo-1-propenyl]benzoic acid,
- 6-[(5,6,7,8-tetrahydro-5,5,8,8-tetramethyl-2-naphthalenyl)ethynyl]-3-pyridinecarboxylic acid,
- 2-(5,6,7,8-tetrahydro-5,5,8,8-tetramethyl-2-naphthyl)-6-benzo(b)thiophenecarboxylic acid,
- 2-(5,6,7,8-tetrahydro-5,5,8,8-tetramethyl-2-naphthyl)-6-benzo(b)furancarboxylic acid,
- 2-(5,6,7,8-tetrahydro-5,5,8,8-tetramethyl- 2-naphthyl)-6-indolecarboxylic acid,
- 2-[3-(1-adamantyl)-4-methoxyphenyl]-5-benzimidazolecarboxylic acid,
- 2-(5,6,7,8-tetrahydro-5,5,8,8-tetramethyl-2-naphthyl)-5-benzimidazolecarboxylic acid,
- 4-(5,6,7,8-tetrahydro-5,5,8,8-tetramethyl-2-anthracenyl)benzoic acid,
- 6-[3-(1-adamantyl)-4-methoxyphenyl]-2-naphthoic acid,
- 4-[3-(1-adamantyl)-4-methoxybenzamido]benzoic acid,
- 4-[3-(1-adamantyl)-4-methoxybenzoylthio]benzoic acid,
- 4-[3-(1-adamantyl)-4-methoxybenzoyloxy]benzoic acid,
- 2-(5,6,7,8-tetrahydro-5,5,8,8-tetramethyl-2-naphthyl)-6-carbonylnaphthalenecarboxylic acid,
- p-(5,6,7,8-tetrahydro-5,5,8,8-tetramethyl-2-1H-benz[f]indolyl) benzoic acid,
- trans-(5,5,8,8-tetramethyl-5,6,7,8-tetrahydro-2-naphthyl)-4-carbonyl-α-methylcinnamic acid,
- vitamin A or retinol, as well as its esters, such as retinol acetate, propionate or palmitate
- vitamin A aldehyde or retinal
- 4-[3-(1-adamantyl)-4-methoxybenzoyloxy]-2-fluorobenzoic acid
- 4-[3-(1-adamantyl)-4-methoxybenzoyloxy]-2-methylbenzoic acid
- 4-[3-(1-adamantyl)-4-methoxybenzoyloxy]-2-hydroxybenzoic acid
- 4-[5-(1-adamantyl)-2-fluoro-4-methoxybenzoyloxy]benzoic acid
- 4-[3,5-di-tert-butyl-4-hydroxybenzoyloxy]benzoic acid
- 4-[3-(1-adamantyl)-4-vinylbenzoyloxy]benzoic acid
- 4-[3-(1-adamantyl)-4-ethylbenzoyloxy]benzoic acid
- 4-[3-(1-adamantyl)-4-allyloxybenzoyloxy]benzoic acid
- 4-[3-(1-adamantyl)-4-methylthiobenzoyloxy]benzoic acid
- 4-(5,6,7,8-tetrahydro-5,5,8,8-tetramethyl-2-naphthylglyoxyloyloxy) benzoic acid
- 4-(5,6,7,8-tetrahydro-5,5,8,8-tetramethyl-2-naphthoyloxymethyl) benzoic acid
- 4-(3,5-di-tert-butyl-4-hydroxybenzoyloxymethyl) benzoic acid
- 4-(3-tert-butyl-4-methoxybenzoyloxymethyl)benzoic acid
- 5-(5,6,7,8-tetrahydro-5,5,8,8-tetramethyl-2-naphthoyloxymethyl)-2-thiophenecarboxylic acid
- 4-(5,6,7,8-tetrahydro-5,5,8,8-tetramethyl-2-naphthoylmethyloxy) benzoic acid
- 4-[1-(5,6,7,8-tetrahydro-5,5,8,8-tetramethyl-2-naphthoyloxy) ethyl] benzoic acid
- 4-[[(5,6,7,8-tetrahydro-5,5,8,8-tetramethyl-2-naphthyl)ethyloxy]carbonyl]benzoic acid
- 4-(5,6,7,8-tetrahydro-5,5,8,8-tetramethyl-2-naphthoylmethylamino)benzoic acid
- 4-(5,6,7,8-tetrahydro-5,5,8,8-tetramethyl-2-naphthoylformamido)benzoic acid
- 4-(α-hydroxy-5,6,7,8-tetrahydro-5,5,8,8-tetramethyl-2-naphthylacetamido)benzoic acid
- 4-(α-fluoro-5,6,7,8-tetrahydro-5,5,8,8-tetramethyl-2-naphthylacetamido)benzoic acid
- 6-[3-(1-adamantyl)-4-(2,3-dihydroxypropyloxy)phenyl]-2-naphthoic acid
- 6-[3-(1-adamantyl)-4-(3-hydroxypropyloxy)phenyl]-2-naphthoic acid
- 6-[3-(1-adamantyl)-4-acetoxymethylphenyl]-2-naphthoic acid

- 6-[3-(1-adamantyl)-4-methoxycarbonylphenyl]-2-naphthoic acid
- 6-[3-(1-adamantyl)-4-methoxycarbonylethylphenyl]-2-naphthoic acid
- 6-[3-(1-adamantyl)-4-(2-hydroxypropyl)phenyl]-2-naphthoic acid
- 2-hydroxy-4-[2-hydroxy-2-(5,6,7,8-tetrahydro-5,5,8,8-tetramethyl-2-naphthyl)ethoxy]benzoic acid
- methyl 2-hydroxy-4-[2-hydroxy-2-(5,6,7,8-tetra-hydro-5,5,8,8-tetramethyl-2-naphthyl)ethoxy]benzoate
- 2-hydroxy-4-[2-hydroxyimino-2-(5,6,7,8-tetrahydro-5,5,8,8-tetramethyl-2-naphthyl)ethoxy]benzoic acid
- 2-acetyloxy-4-[2-acetyloxy-2-(5,6,7,8-tetrahydro-5,5,8,8-tetramethyl-2-naphthyl)ethoxy]benzoic acid
- 2-hydroxy-4-[2-acetyloxy-2-(5,6,7,8-tetrahydro-5,5,8,8-tetramethyl-2-naphthyl)ethoxy]benzoic acid
- 2-acetyloxy-4-[2-hydroxy-2-(5,6,7,8-tetrahydro-5,5,8,8-tetramethyl-2-naphthyl)ethoxy]benzoic acid
- 4-(N-methyl-5,6,7,8-tetrahydro-5,5.8,8-tetramethyl-2 -naphthylcarboxamidino)benzoic acid
- 4-(5,6,7,8-tetrahydro-5,5,8,8-tetramethyl-2-naphthylcarboxamidino)benzoic acid.

**3.** Composition according to Claim 1 or 2, characterized by the fact that the said inhibitory sterol is chosen from compounds having the hydrocarbon backbone of cholesterol or lanosterol and carrying, in addition, at least one alkylated, oxygenated and/or nitrogenous substituent, and/or at least one unsaturation.

**4.** Composition according to any one of the preceding claims, characterized by the fact that the said sterol which inhibits the synthesis of cholesterol is an inhibitor of 3-hydroxy-3-methylglutarylcoenzyme A reductase.

**5.** Composition according to Claim 4, characterized by the fact that the said inhibitory sterol is chosen from sterols carrying, on the cholesterol structure or on a derived structure, at least one oxygenated substituent other than the hydroxyl in position 3, and 6-nitro-cholesterol.

**6.** Composition according to Claim 5, characterized by the fact that the oxygenated substituent is a hydroxyl, an oxo, an epoxy, a peroxide or a carbonyl in position 4, 7, 14, 15, 20, 22, 24, 25 or 26.

**7.** Composition according to Claim 6, characterized by the fact that the said sterol is 25-hydroxycholesterol.

**8.** Composition according to any one of Claims 1 to 3, characterized by the fact that the said inhibitor of the biosynthesis of cholesterol is an inhibitor of 2,3-oxydosqualene cyclase.

**9.** Composition according to Claim 8, characterized by the fact that the said sterol is chosen from 2-aza-2,3-dihydrosqualene or its derivatives, and N-[(1,5,9)- trimethyldecyl]-4$\alpha$,10-dimethyl-8-aza-transdecal-3$\beta$-ol.

**10.** Composition according to any one of Claims 1 to 3, characterized by the fact that the said sterol is an inhibitor of the metabolism of 24,25-dihydrolanosterol.

**11.** Composition according to Claim 10, characterized by the fact that the said sterol is chosen from:
- 7-oxolanost-8-en-3$\beta$-ol,
- 3$\beta$-acetoxylanost-8-en-7-one,
- 7-oxolanost-5,8,11-trien-3$\beta$-ol.

**12.** Composition according to any one of Claims 1 to 3, characterized by the fact that the said sterol is an inhibitor of the conversion of lanosterol to cholesterol, and in particular of the conversion of lanosterol to desmosterol.

**13.** Composition according to any one of Claims 1 to 3, characterized by the fact that the said sterol is an inhibitor of the conversion of desmosterol to cholesterol.

**14.** Composition according to Claim 12 or 13, characterized by the fact that the said sterol is chosen from 22-oximinocholesterol and 20,25-diazacholesterol.

**15.** Composition according to any one of the preceding claims, characterized by the fact that the retinoid is present in proportions which may range from 0.0001 to 50% by weight relative to the total weight of the composition.

**16.** Composition according to Claim 15, characterized by the fact that the retinoid is present in proportions which may range from 0.0001 to 1% by weight relative to the total weight of the composition.

17. Composition according to Claim 15, characterized by the fact that the retinoid is present in proportions which may range from 0.01 to 50% by weight relative to the total weight of the composition.

18. Composition according to either of Claims 16 and 17, characterized by the fact that the ratio of the molar concentration of the sterol to the molar concentration of the retinoid is in the range from 0.1 to 10.

19. Composition according to any one of the preceding claims, characterized by the fact that it contains, in addition to the active ingredients, a pharmaceutical vehicle compatible with a topical (skin and mucous membranes), ocular, buccal or systemic administration.

20. Composition according to any one of the preceding claims, characterized by the fact that it is provided in the form of a composition in two parts comprising, combined in an appropriate packaging, on the one hand, a retinoid as defined in either of Claims 1 and 2, in an appropriate container, and, in a second container, a sterol which inhibits the biosynthesis of cholesterol as defined in any one of Claims 3 to 14.

21. Use, as a combination, of at least one retinoid and of at least one sterol, in the preparation of a medicinal product for treating or for correcting disorders of epidermal keratinization, any other disorder or any other functional defect or excess of epidermal or epithelial proliferation, and/or disorders of sebaceous function, accompanied or not accompanied by an inflammatory and/or immunoallergic component, the said medicinal product being as defined in any one of Claims 1 to 20.

**Claims for the following Contracting states : ES, GR**

1. Process for the preparation of a pharmaceutical or cosmetic composition, characterized by the fact that the following are used as active ingredients,
   - at least one retinoid capable of inhibiting the expression of membrane transglutaminase,
   - and at least one sterol which inhibits the biosynthesis of cholesterol, and that a vehicle compatible with a topical, ocular or systemic administration is added to the said active ingredients.

2. Process according to Claim 1, characterized by the fact that the said retinoid is chosen from:
   - all-trans- or 13-cis-retinoic acid,
   - (all-E)-9-(4-methoxy-2,3,6-trimethylphenyl)-3,7-dimethyl-2,4,6,8-nonatetraenoic acid,
   - (E,E,E)-7-(2,3-dihydro-1,1,3,3-tetramethyl-1H-inden-5-yl)-3,7-dimethyl-2,4,6-octatrienoic acid,
   - (E,E,E)-7-(5,6,7,8-tetrahydro-5,5,8,8-tetramethyl-2-naphthalenyl-3,7-dimethyl-2,4,6-octatrienoic acid,
   - (E)-4-[(2,3-dihydro-1,1,3,3-tetramethyl-1H-inden-5-yl)-1-propenyl]benzoic acid,
   - (E)-4-[2-(5,6,7,8-tetrahydro-5,5,8,8-tetramethyl-2-naphthalenyl)-1-propenyl]benzoic acid,
   - 2-(5,6,7,8-tetrahydro-5,5,8,8-tetramethyl-2-naphthyl)-6-naphthalenecarboxylic acid,
   - (E)-4-[2-(5,6,7,8-tetrahydro-5,5,8,8-tetramethyl-2-naphthalenyl)-1-propenyl] benzenesulphonic acid,
   - (E,E)-4-[2-methyl-4-(2,6,6-trimethyl-1-cyclohexen-1-yl)-1,3-butadienyl]benzoic acid,
   - (E,E)-4-[4-methyl-6-(2,6,6-trimethyl-1-cyclohexen-1-yl)-1,3,5-hexatrienyl]benzoic acid,
   - (E)-6-[2-(2,6,6-trimethyl-1-cyclohexen-1-yl)-ethenyl]-2-naphthalenecarboxylic acid,
   - (E,E)-5-[2-methyl-4-(2,6,6-trimethyl-1-cyclohexen-1-yl)1,3-butadienyl]-2-thiophenecarboxylic acid,
   - (E)-4-[2-(5,6,7,8-tetrahydro-8,8-dimethyl-2-naphthalenyl)-1-propenyl]benzoic acid,
   - 4-[(5,6,7,8-tetrahydro-5,5,8,8-tetramethyl-2-naphthalenyl)ethynyl]benzoic acid,
   - (E)-4-[2-(5,6,7,8-tetrahydro-3-methyl-5,5,8,8-tetramethyl-2-naphthalenyl)-1-propenyl]benzoic acid,
   - 4-(5,6,7,8-tetrahydro-5,5,8,8-tetramethyl-2-naphthalenylcarbamoyl)benzoic acid,
   - 4-(5,6,7,8-tetrahydro-5,5,8,8-tetramethyl-2-naphthamido)benzoic acid,
   - (E)-4-[3-(3,5-di-tert-butylphenyl)-3-oxo-1-propenyl]benzoic acid,
   - 6-[(5,6,7,8-tetrahydro-5,5,8,8-tetramethyl-2-naphthalenyl)ethynyl]-3-pyridinecarboxylic acid,
   - 2-(5,6,7,8-tetrahydro-5,5,8,8-tetramethyl-2-naphtyl)-6-benzo(b)thiophenecarboxylic acid,
   - 2-(5,6,7,8-tetrahydro-5,5,8,8-tetramethyl-2-naphthyl)-6-benzo(b)furancarboxylic acid,
   - 2-(5,6,7,8-tetrahydro-5,5,8,8-tetramethyl-2-naphthyl)-6-indolecarboxylic acid,
     2-[3-(1-adamantyl-4-methoxyphenyl]-5-benzimidazolecarboxylic acid,
   - 2-(5,6,7,8-tetrahydro-5,5,8,8-tetramethyl-2-naphthyl)-5-benzimidazolecarboxylic acid,
   - 4-(5,6,7,8-tetrahydro-5,5,8,8-tetramethyl-2-anthracenyl)benzoic acid,
   - 6-[3-(1-adamantyl)-4-methoxyphenyl]-2-naphthoic acid,
   - 4-[3-(1-adamantyl)-4-methoxybenzamido]benzoic acid,
   - 4-[3-(1-adamantyl)-4-methoxybenzoylthio]benzoic acid,

- 4-[3-(1-adamantyl)-4-methoxybenzoyloxy]benzoic acid,
- 2-(5,6,7,8-tetrahydro-5,5,8,8-tetramethyl-2-naphthyl)-6-carbonylnaphthalenecarboxylic acid,
- p-(5,6,7,8-tetrahydro-5,5,8,8-tetramethyl-2-1H-benz[f]indolyl)benzoic acid,
- trans-(5,5,8,8-tetramethyl-5,6,7,8-tetrahydro-2-naphthyl)-4-carbonyl-$\alpha$-methylcinnamic acid,
- vitamin A or retinol, as well as its esters, such as retinol acetate, propionate or palmitate
- vitamin A aldehyde or retinal
- 4-[3-(1-adamantyl)-4-methoxybenzoyloxy]-2-fluorobenzoic acid
- 4-[3-(1-adamantyl)-4-methoxybenzoyloxy]-2-methylbenzoic acid
- 4-[3-(1-adamantyl)-4-methoxybenzoyloxy]-2-hydroxybenzoic acid
- 4-[5-(1-adamantyl)-2-fluoro-4-methoxybenzoyloxy]benzoic acid
- 4-[3,5-di-tert-butyl-4-hydroxybenzoyloxy]benzoic acid
- 4-[3-(1-adamantyl)-4-vinylbenzoyloxy]benzoic acid
- 4-[3-(1-adamantyl)-4-ethylbenzoyloxy]benzoic acid
- 4-[3-(1-adamantyl)-4-allyloxybenzoyloxy]benzoic acid
- 4-[3-(1-adamantyl)-4-methylthiobenzoyloxy]benzoic acid
- 4-(5,6,7,8-tetrahydro-5,5,8,8-tetramethyl-2-naphthylglyoxyloyloxy) benzoic acid
- 4-(5,6,7,8-tetrahydro-5,5,8,8-tetramethyl-2-naphthoyloxymethyl)benzoic acid
- 4-(3,5-di-tert-butyl-4-hydroxybenzoyloxymethyl)benzoic acid benzoic acid
- 4-(3-tert-butyl-4-methoxybenzoyloxymethyl)benzoic acid
- 5-(5,6,7,8-tetrahydro-5,5,8,8-tetramethyl-2-naphthoyloxymethyl)-2-thiophenecarboxylic acid
- 4-(5,6,7,8-tetrahydro-5,5,8,8-tetramethyl-2-naphthoylmethyloxy) benzoic acid
- 4-[1-(5,6,7,8-tetrahydro-5,5,8,8-tetramethyl-2-naphthoyloxy)ethyl]benzoic acid
- 4-[[(5,6,7,8-tetrahydro-5,5,8,8,tetramethyl-2-naphthyl)ethyloxy]carbonyl]benzoic acid
- 4-(5,6,7,8-tetrahydro-5,5,8,8-tetramethyl-2-naphthoylmethylamino)benzoic acid
- 4-(5,6,7,8-tetrahydro-5,5,8,8-tetramethyl-2-naphthoylformamido)benzoic acid
- 4-($\alpha$-hydroxy-5,6,7,8-tetrahydro-5,5,8,8-tetramethyl-2-naphthylacetamido)benzoic acid
- 4-($\alpha$-fluoro-5,6,7,8-tetrahydro-5,5,8,8-tetramethyl-2-naphthylacetamido)benzoic acid
- 6-[3-(1-adamantyl)-4-(2,3-dihydroxypropyloxy)phenyl]-2-naphthoic acid
- 6-[3-(1-adamantyl)-4-(3-hydroxypropyloxy)phenyl]-2-naphthoic acid
- 6-[3-(1-adamantyl)-4-acetoxymethylphenyl]-2-naphthoic acid
- 6-[3-(1-adamantyl)-4-methoxycarbonylphenyl]-2-naphthoic acid
- -6-[3-(1-adamantyl)-4-methoxycarbonylethylphenyl]-2-naphthoic acid
- -6-[3-(1-adamantyl)-4-(2-hydroxypropyl)phenyl]-2-naphthoic acid,
- 2-hydroxy-4-[2-hydroxy-2-(5,6,7,8-tetrahydro-5,5,8,8-tetramethyl-2-naphthyl)ethoxy]benzoic acid
- methyl 2-hydroxy-4-[2-hydroxy-2- (5,6,7,8-tetra-hydro-5,5,8,8-tetramethyl-2-naphthyl)ethoxy]benzoate
- 2-hydroxy-4-[2-hydroxyimino-2-(5,6,7,8-tetrahydro-5,5,8,8-tetramethyl-2-naphthyl)ethoxy]benzoic acid
- 2-acetyloxy-4-[2-acetyloxy-2-(5,6,7,8-tetrahydro-5,5,8,8-tetramethyl-2-naphthyl)ethoxy]benzoic acid
- 2-hydroxy-4-[2-acetyloxy-2-(5,6,7,8-tetrahydro-5,5,8,8-tetramethyl-2-naphthyl)ethoxy]benzoic acid
- 2-acetyloxy-4-[2-hydroxy-2-(5,6,7,8-tetrahydro-5,5,8,8-tetramethyl-2-naphthyl)ethoxy]benzoic acid
- 4-(N-methyl-5,6,7,8-tetrahydro-5,5,8,8-tetramethyl-2-naphthylcarboxamidino)benzoic acid
- 4-(5,6,7,8-tetrahydro-5,5,8,8-tetramethyl-2-naphthylcarboxamidino)benzoic acid.

3. Process according to Claim 1 or 2, characterised by the fact that the said inhibitory sterol is chosen from compounds having the hydrocarbon backbone of cholesterol or lanosterol and carrying, in addition, at least one alkylated, oxygenated and/or nitrogenous substituent, and/or at least one unsaturation.

4. Process according to any one of the preceding claims, characterized by the fact that the said sterol which inhibits the synthesis of cholesterol is an inhibitor of 3-hydroxy-3-methylglutarylcoenzyme A reductase.

5. Process according to Claim 4, characterized by the fact that the said inhibitory sterol is chosen from sterols carrying, on the cholesterol structure or on a derived structure, at least one oxygenated substituent other than the hydroxyl in position 3, and 6-nitro-cholesterol.

6. Process according to Claim 5, characterized by the fact that the oxygenated substituent is a hydroxyl, an oxo, an epoxy, a peroxide or a carbonyl in position 4, 7, 14, 15, 20, 22, 24, 25 or 26.

7. Process according to Claim 6, characterized by the fact that the said sterol is 25-hydroxycholesterol.

8. Process according to any one of Claims 1 to 3, characterized by the fact that the said inhibitor of the biosynthesis of cholesterol is an inhibitor of 2,3-oxydosqualene cyclase.

**9.** Process according to Claim 8, characterized by the fact that the said sterol is chosen from 2-aza-2,3-dihydrosqualene or its derivatives, and N-[(1,5,9)-trimethyldecyl]-4α,10-dimethyl-8-aza-transdecal-3β-ol.

**10.** Process according to any one of Claims 1 to 3, characterized by the fact that the said sterol is an inhibitor of the metabolism of 24,25-dihydrolanosterol.

**11.** Process according to Claim 10, characterized by the fact that the said sterol is chosen from:
- 7-oxolanost-8-en-3β-ol,
- 3β-acetoxylanost-8-en-7-one,
- 7-oxolanost-5,8,11-trien-3β-ol.

**12.** Process according to any one of Claims 1 to 3, characterized by the fact that the said sterol is an inhibitor of the conversion of lanosterol to cholesterol, and in particular of the conversion of lanosterol to desmosterol.

**13.** Process according to any one of Claims 1 to 3, characterized by the fact that the said sterol is an inhibitor of the conversion of desmosterol to cholesterol.

**14.** Process according to Claim 12 or 13, characterized by the fact that the said sterol is chosen from 22-oximinocholesterol and 20,25-diazacholesterol.

**15.** Process according to any one of the preceding claims, characterized by the fact that the retinoid is used in proportions which may range from 0.0001 to 50% by weight relative to the total weight of the composition.

**16.** Process according to Claim 15, characterized by the fact that the retinoid is used in proportions which may range from 0.0001 to 1% by weight relative to the total weight of the composition.

**17.** Process according to Claim 15, characterized by the fact that the retinoid is used in proportions which may range from 0.01 to 50% by weight relative to the total weight of the composition.

**18.** Process according to either of Claims 16 and 17, characterized by the fact that the ratio of the molar concentration of the sterol to the molar concentration of the retinoid is in the range from 0.1 to 10.

**19.** Process according to any one of the preceding claims, characterized by the fact that the composition is prepared in the form of composition in two parts comprising, combined in an appropriate packaging, on the one hand, a retinoid as defined in either of Claims 1 and 2, in an appropriate container, and, in a second container, a sterol which inhibits the biosynthesis of cholesterol as defined in any one of Claims 3 to 14.

**20.** Use, as a combination, of at least one retinoid and of at least one sterol, in the preparation of a pharmaceutical composition for treating or for correcting disorders af epidermal keratinization, disorders of epidermal or epithelial proliferation, and/or disorders of sebaceous function, accompanied or not accompanied by an inflammatory and/or immunoallergic component, or in the preparation of a cosmetic composition for combating skin ageing or improving the appearance of the skin in people with disorders of keratinization or those suffering from seborrhoea, the said composition being as defined in any one of Claims 1 to 19.